# EUROPÄISCHE PATENTSCHRIFT

(11) **EP 2 705 037 B1**
(45) Veröffentlichungstag und Bekanntmachung des Hinweises auf die Patenterteilung: **22.06.2016**
(21) Anmeldenummer: 12717331.8
(22) Anmeldetag: 02.05.2012
(51) Int. Cl.: C07D 487/04, A61K 31/519, A61K 31/5365

(54) **SUBSTITUIERTE IMIDAZOPYRIDINE UND IMIDAZOPYRIDAZINE UND IHRE VERWENDUNG**
SUBSTITUTED IMIDAZOPYRIDINES AND IMIDAZOPYRIDAZINES AND USE THEREOF
IMIDAZOPYRIDAZINES SUBSTITUÉES ET IMIDAZOPYRIDAZINES, ET LEUR UTILISATION

(30) Priorität: 06.05.2011 DE 102011075399; 11.01.2012 DE 102012200356
(43) Veröffentlichungstag der Anmeldung: 12.03.2014
(73) Patentinhaber: Bayer Intellectual Property GmbH, 40789 Monheim (DE); Bayer Pharma Aktiengesellschaft, 13353 Berlin (DE)
(72) Erfinder: FOLLMANN, Markus, 50589 Köln (DE); STASCH, Johannes-Peter, 42651 Solingen (DE); REDLICH, Gorden, 44799 Bochum (DE); GRIEBENOW, Nils, 41541 Dormagen (DE); LANG, Dieter, 42553 Velbert (DE); WUNDER, Frank, 42117 Wuppertal (DE); LI, Volkhart Min-Jian, 42553 Velbert (DE)
(74) Vertreter: BIP Patents
(86) Internationale Anmeldenummer: PCT/EP2012/058046
(87) Internationale Veröffentlichungsnummer: WO 2012/152629

(56) Entgegenhaltungen:
- WO-A1-2010/065275
- WO-A1-2011/149921
- DE-A1- 19 834 044
- WUNDER F ET AL: "A cell-based cGMP assay useful for ultra-high-throughput screening and identification of modulators of the nitric oxide/cGMP pathway", ANALYTICAL BIOCHEMISTRY, ACADEMIC PRESS INC, NEW YORK, Bd. 339, Nr. 1, 1. April 2005 (2005-04-01) , Seiten 104-112, XP004781305, ISSN: 0003-2697, DOI: 10.1016/J.AB.2004.12.025

## Beschreibung

Die vorliegende Anmeldung betrifft neue substituierte Imidazopyridine und Imidazopyridazine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart. WO 2008/031513 beschreibt unter anderem substituierte Imidazopyridne und Imidazopyrimidine als Stimulatoren der löslichen Guanylatcyclase. 4-Amino-5,5-dimethyl-5,7,dihydro-6H-pyrrolo[2,3-d]pyrimidone mit Imidazopyridin und -pyrimidin-Substituenten als sGC Aktivatoren werden in WO 2010/065275 und WO 2011/149921 offenbart.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Gegenstand der vorliegenden Erfindung sind Verbindungen der allgemeinen Formel (I) 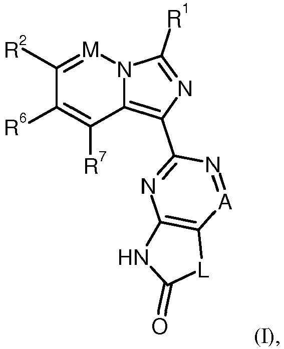 wie gemäß Anspruch 1 definiert.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸² Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

In der Formel der Gruppe, für die L stehen kann, steht der Endpunkt der Linie, an dem das Zeichen # bzw. ## steht, nicht für ein Kohlenstoffatom beziehungsweise eine CH₂-Gruppe, sondern ist Bestandteil der Bindung zu dem jeweils bezeichneten Atom, an das L gebunden ist.

Die Verbindungen der Formel (I-1) bilden eine Untergruppe der Verbindungen der Formel (I), in welchen R⁶ und R⁷ für Wasserstoff stehen.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.

Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-diyl, Propan-1,2-diyl, Propan-2,2-diyl, Butan-1,4-diyl, Butan-1,2-diyl, Butan-1,3-diyl und Butan-2,3-diyl.

Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy,

Alkoxycarbonyl steht im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.

Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.

Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, N-tert.-Butyl-*N*-methylamino, *N*-Ethyl-*N*-n-pentylamino und *N*-n-Hexyl-*N*-methylamino.

Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.

5- oder 6-gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.

Halogen steht im Rahmen der Erfindung für Fluor, Chlor, Brom und Iod. Bevorzugt sind Brom und Iod.

Eine Oxo-Gruppe steht im Rahmen der Erfindung für ein Sauerstoffatom, das über eine Doppelbindung an ein Kohlenstoffatom gebunden ist.

Eine Thiooxo-Gruppe steht im Rahmen der Erfindung für ein Schwefelatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Gegenstand der vorliegenden Erfindung Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
- M: für CH oder N steht,
- R¹: für 4,4,4-Trifluorbut-1-yl, 3,3,4,4-Tetrafluorbut-1-yl, 3,3,4,4,4-Pentafluorbut-1-yl oder Benzyl steht,
wobei Benzyl mit 1 bis 3 Substituenten Fluor substituiert ist,
- R²: für Fluor oder Chlor steht, wenn M für CH steht,
oder
- R²: für Wasserstoff steht, wenn M für N steht,
- R⁶: für Wasserstoff steht,
- R⁷: für Wasserstoff steht,
sowie ihre Salze, Solvate und Solvate der Salze.

Bevorzugt sind im Rahmen der vorliegenden Erfindung Verbindungen der Formel (I-1), in welcher 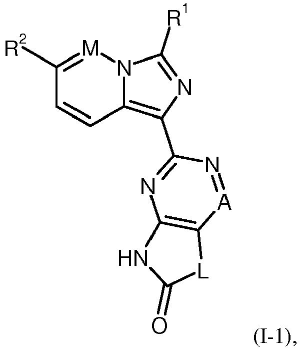 in welcher
- A: für Stickstoff oder CR³ steht,
wobei
R³ für Wasserstoff, Deuterium, Fluor, Difluormethyl, Trifluormethyl, (C₁-C₄)-Alkyl, Cyclopropyl oder Cyclobutyl steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl, Hydroxy oder Amino steht,
R^{4B} für Wasserstoff, Fluor, (C₁-C₄)-Alkyl, Trifluormethyl, (C₁-C₄)-Alkoxycarbonyl-amino oder Phenyl steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
- R^{4A} und R^{4B}: zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
worin der 3- bis 6-gliedrige Carbocyclus und der 4- bis 6-gliedrige Heterocyclus mit 1 oder 2 Substituenten unabhängig voneinander ausgewählt aus der Gruppe Fluor und (C₁-C₄)-Alkyl substituiert sein können,
oder
- R^{4A} und R^{4B}: zusammen mit dem Kohlenstoffatom, an das sie gebunden sind, eine (C₂-C₄)-Alkenyl-Gruppe bilden,
- R^{5A}: für Wasserstoff, Fluor, (C₁-C₄)-Alkyl oder Hydroxy steht,
- R^{5B}: für Wasserstoff, Fluor, (C₁-C₄)-Alkyl oder Trifluormethyl steht,
- M: für CH oder N steht,
- R¹: für (C₁-C₆)-Alkyl oder Benzyl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
und
wobei Benzyl mit 1 bis 3 Substituenten Fluor substituiert ist,
- R²: für Wasserstoff, Fluor oder Chlor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I-1), in welcher
- A: für Stickstoff oder CR³ steht,
wobei
R³ für Wasserstoff, Fluor, Difluormethyl, Trifluormethyl, Methyl, Ethyl oder Cyclopropyl steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 oder 1 steht,
R^{4A} für Wasserstoff, Fluor, Methyl, Ethyl oder Hydroxy steht,
R^{4B} für Wasserstoff, Fluor, Methyl, Ethyl, Trifluormethyl, Methoxycarbonylamino oder Phenyl steht,
worin Methyl und Ethyl mit 1 bis 3 Substituenten unabhängig voneinander ausgewählt aus der Gruppe Fluor, Trifluormethyl oder Hydroxy substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- M: für CH steht,
- R¹: für 2,2,2-Trifluorethyl, 3,3,3-Trifluorprop-1-yl, 4,4,4-Trifluorbut-1yl, 3,3,4,4,4-Pentafluorbut-1yl oder Benzyl steht,
wobei Benzyl mit 1 bis 3 Substituenten Fluor substituiert ist,
- R²: für Fluor oder Chlor steht,
sowie ihre Salze, Solvate und Solvate der Salze.

Bevorzugt sind im Rahmen der vorliegenden Erfindung auch Verbindungen der Formel (I-1), in welcher
- A: für Stickstoff oder CR³ steht,
wobei
R³ für Wasserstoff, Fluor, Difluormethyl, Trifluormethyl, Methyl, Ethyl oder Cyclopropyl steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 oder 1 steht,
R^{4A} für Wasserstoff, Fluor, Methyl, Ethyl oder Hydroxy steht,
R^{4B} für Wasserstoff, Fluor, Methyl, Ethyl, Trifluormethyl, Methoxycarbonylamino oder Phenyl steht,
worin Methyl und Ethyl mit 1 bis 3 Substituenten unabhängig voneinander ausgewählt aus der Gruppe Fluor, Trifluormethyl oder Hydroxy substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- M: für N steht,
- R¹: für 2,2,2-Trifluorethyl, 3,3,3-Trifluorprop-1-yl, 4,4,4-Trifluorbut-1yl, 3,3,4,4,4-Pentafluorbut-1yl oder Benzyl steht,
wobei Benzyl mit 1 bis 3 Substituenten Fluor substituiert ist,
- R²: für Wasserstoff steht,
sowie ihre Salze, Solvate und Solvate der Salze.

Besonders bevorzugt sind im Rahmen der vorliegenden Erfindung Verbindungen der Formel (I-1), in welcher
- A: für Stickstoff oder CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- M: für CH steht,
- R¹: für 3,3,4,4,4-Pentafluorbut-1yl, 2-Fluorbenzyl oder 2,3,6-Trifluorbenzyl steht,
- R²: für Fluor oder Chlor steht,
sowie ihre Salze, Solvate und Solvate der Salze.

Besonders bevorzugt sind im Rahmen der vorliegenden Erfindung Verbindungen der Formel (I-1), in welcher
- A: für Stickstoff oder CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden, worin der Cyclopropyl- und der Cyclobutyl-Ring mit 1 oder 2 Substituenten unabhängig voneinander ausgewählt aus der Gruppe Fluor und Methyl substituiert sein können,
- M: für N steht,
- R¹: für 3,3,4,4,4-Pentafluorbut-1yl, 2-Fluorbenzyl oder 2,3,6-Trifluorbenzyl steht,
- R²: für Wasserstoff steht,
sowie ihre Salze, Solvate und Solvate der Salze.

Bevorzugt sind im Rahmen der vorliegenden Erfindung auch Verbindungen der Formel (I) und (I-1), in welcher
- A: für Stickstoff steht,
sowie ihre Salze, Solvate und Solvate der Salze.

Bevorzugt sind im Rahmen der vorliegenden Erfindung auch Verbindungen der Formel (I) und (I-1), in welcher
- A: für CR³ steht,
wobei
R³ für Wasserstoff steht,
sowie ihre Salze, Solvate und Solvate der Salze.

Bevorzugt sind im Rahmen der vorliegenden Erfindung auch Verbindungen der Formel (I) und (I-1), in welcher
- M: für N steht,
- R²: für Wasserstoff steht,
steht, sowie ihre Salze, Solvate und Solvate der Salze.

Bevorzugt sind im Rahmen der vorliegenden Erfindung auch Verbindungen der Formel (I) und (I-1), in welcher
- M: für CH steht,
- R²: für Fluor oder Chlor steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl, Trifluormethyl, 2,2,2-Trifluorethyl oder 1,1,2,2,2-Pentafluorethyl steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Trifluormethyl, 2,2,2-Trifluorethyl oder 1,1,2,2,2-Pentafluorethyl steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Methyl steht,
R^{4B} für Methyl steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
sowie ihre Salze, Solvate und Solvate der Salze.

Bevorzugt sind im Rahmen der vorliegenden Erfindung auch Verbindungen der Formel (I), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Azetidinyl-, Pyrrolidinyl-, Tetrahydrofuranyl-, Piperidinyl- oder Tetrahydropyranyl-Ring bilden,
sowie ihre Salze, Solvate und Solvate der Salze.

Bevorzugt sind im Rahmen der vorliegenden Erfindung auch Verbindungen der Formel (I) und (I-1), in welcher
- R¹: für Benzyl steht,
wobei Benzyl mit 1 bis 3 Substituenten Fluor substituiert ist,
sowie ihre Salze, Solvate und Solvate der Salze.

Bevorzugt sind im Rahmen der vorliegenden Erfindung auch Verbindungen der Formel (I) und (I-1), in welcher
- R¹: für 2-Fluorbenzyl, 2,3-Difluorbenzyl oder 2,3,6-Trifluorbenzyl steht,
sowie ihre Salze, Solvate und Solvate der Salze.

Bevorzugt sind im Rahmen der vorliegenden Erfindung auch Verbindungen der Formel (I) und (I-1), in welcher
- R¹: für 2,2,2-Trifluorethyl, 3,3,3-Trifluorprop-1-yl, 4,4,4-Trifluorbut-1yl oder 3,3,4,4,4-Pentafluorbut-1yl steht,
sowie ihre Salze, Solvate und Solvate der Salze.

Bevorzugt sind im Rahmen der vorliegenden Erfindung auch Verbindungen der Formel (I), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring steht,
p für eine Zahl 0 steht,
R^{4A} für Wasserstoff, Fluor, Methyl, Ethyl, Hydroxy oder Amino steht,
R^{4B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -Q-R⁸ steht, worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
Q für eine Bindung oder Methylen steht,
R⁸ für -(C=O)ᵣ NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Bevorzugt sind im Rahmen der vorliegenden Erfindung auch Verbindungen der Formel (I), in welcher
- A: für N steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl, Ethyl, Hydroxy oder Amino steht,
R^{4B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -Q-R⁸ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
Q für eine Bindung oder Methylen steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Bevorzugt sind im Rahmen der vorliegenden Erfindung auch Verbindungen der Formel (I), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht, sowie ihre Salze, Solvate und Solvate der Salze.

Bevorzugt sind im Rahmen der vorliegenden Erfindung auch Verbindungen der Formel (I), in welcher
- A: für Stickstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
sowie ihre Salze, Solvate und Solvate der Salze.

Bevorzugt sind im Rahmen der vorliegenden Erfindung auch Verbindungen der Formel (I), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring steht,
p für eine Zahl 0 steht,
R^{4A} für Wasserstoff, Fluor, Methyl, Ethyl, Hydroxy oder Amino steht,
R^{4B} für eine Gruppe der Formel -Q-R⁸ steht,
worin
Q für eine Bindung steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxy-carbonyl, Methoxycarbonyl, Ethoxycarbonyl und Amino substituiert sein können,
und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Bevorzugt sind im Rahmen der vorliegenden Erfindung auch Verbindungen der Formel (I), in welcher
- A: für N steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl, Ethyl, Hydroxy oder Amino steht,
R^{4B} für eine Gruppe der Formel -Q-R⁸ steht,
worin
Q für eine Bindung steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombina-tionen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 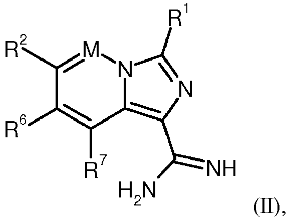 in welcher M, R¹, R², R⁶ und R⁷ jeweils die oben angegebenen Bedeutungen haben,
   in einem inerten Lösungsmittel in Gegenwart einer geeigneten Base mit einer Verbindung der Formel (III) 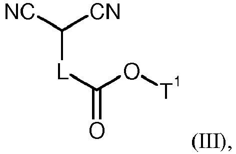 in welcher L die oben angegebene Bedeutung hat und
   T¹ für (C₁-C₄)-Alkyl steht,
   zu einer Verbindung der Formel (IV) 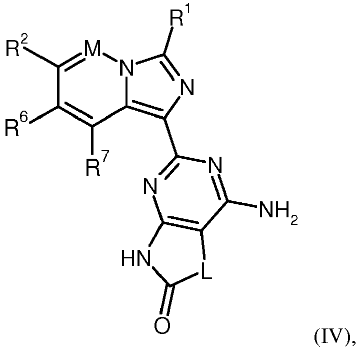 in welcher M, L, R¹, R², R⁶ und R⁷ jeweils die oben angegebenen Bedeutungen haben,
   cyclisiert, diese anschließend in einem inerten Lösungsmittel mit iso-Pentylnitrit und einem Halogen-Äquivalent in eine Verbindung der Formel (I-A) 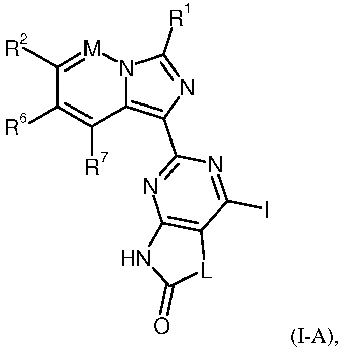 in welcher M, L, R¹, R², R⁶ und R⁷ jeweils die oben angegebenen Bedeutungen haben, überführt,
   oder
[B] eine Verbindung der Formel (I-A) in einen inerten Lösungsmittel in Gegenwart eines geeigneten Übergangsmetallkatalysators zu einer Verbindung der Formel (I-B) 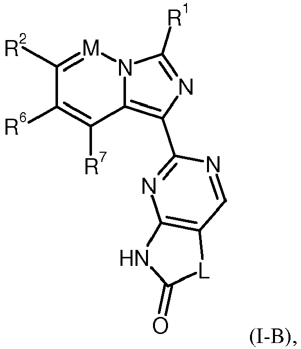 in welcher M, L, R¹, R², R⁶ und R⁷ jeweils die oben angegebenen Bedeutungen haben, umsetzt,
   oder
[C] eine Verbindung der Formel (II) in einem inerten Lösungsmittel in Gegenwart einer geeigneten Base mit einer Verbindung der Formel (V) 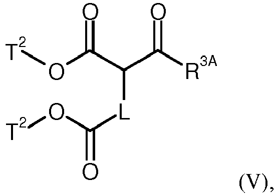
   in welcher L die oben angegebene Bedeutung hat,
   R^{3A} für Wasserstoff, Halogen, (C₁-C₄)-Alkyl oder Hydroxy steht und
   T² für (C₁-C₄)-Alkyl steht,
   zu einer Verbindung der Formel (VI) 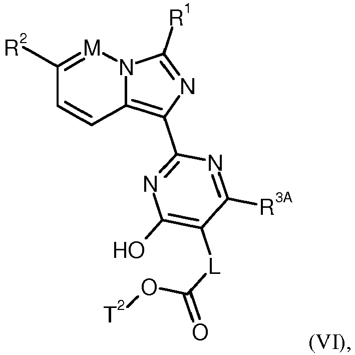 in welcher M, L, R¹, R², R^{3A}, R⁶, R⁷ und T² jeweils die zuvor angegebenen Bedeutungen haben,
   umsetzt, diese anschliessend mit Phosphorylchlorid in eine Verbindung der Formel (VII) 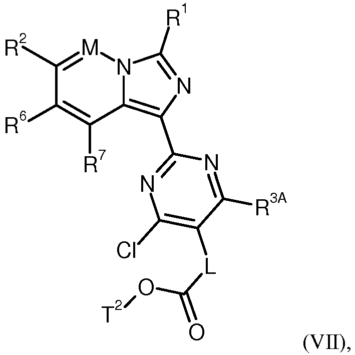 in welcher M, L, R¹, R², R^{3A}, R⁶, R⁷ und T² jeweils die zuvor angegebenen Bedeutungen haben,
   überführt, diese im folgenden in einem inerten Lösungsmittel in eine entsprechende Azid-Verbindung überführt und diese direkt zu einer Verbindung der Formel (VIII) 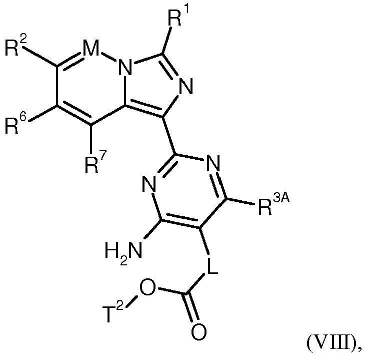 in welcher M, L, R¹, R², R^{3A}, R⁶, R⁷ und T² jeweils die zuvor angegebenen Bedeutungen haben,
   reduziert, und diese dann in einem inerten Lösungsmittel in Gegenwart einer geeigneten Base zu einer Verbindung der Formel (I-C) 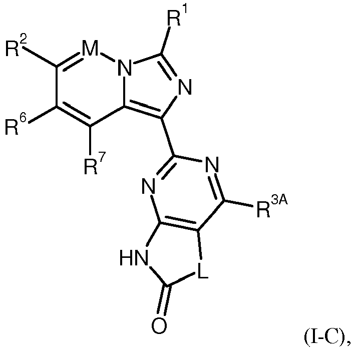 in welcher M, L, R¹, R² und R^{3A}, R⁶, R⁷ jeweils die zuvor angegebenen Bedeutungen haben,
   umsetzt,
   oder
[D] eine Verbindung der Formel (II) in einem inerten Lösungsmittel in Gegenwart einer geeigneten Base mit Hydrazinhydrat zu einer Verbindung der Formel (IX) 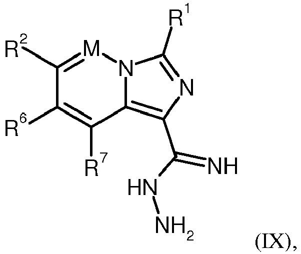 in welcher M, R¹, R², R⁶ und R⁷ jeweils die oben angegebenen Bedeutungen haben, umsetzt, diese dann in einem inerten Lösungsmittel mit einer Verbindung der Formel (X) 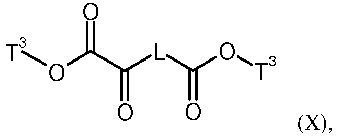
   in welcher L die oben angegebene Bedeutung hat und
   T³ für (C₁-C₄)-Alkyl steht,
   zu einer Verbindung der Formel (XI) 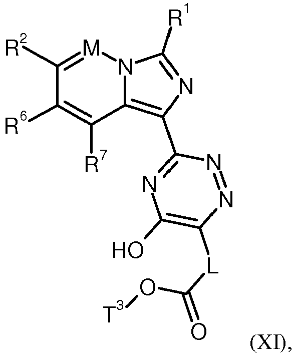 in welcher M, L, R¹, R², R⁶, R⁷ und T³ jeweils die oben angegebenen Bedeutungen haben,
   reagiert, diese anschliessend in eine Verbindung der Formel (XII) 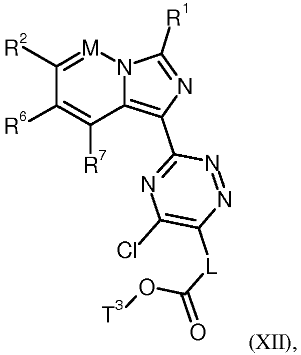 in welcher M, L, R¹, R², R⁶, R⁷ und T³ jeweils die oben angegebenen Bedeutungen haben, überführt, und diese direkt mit Ammoniak zu einer Verbindung der Formel (XIII) 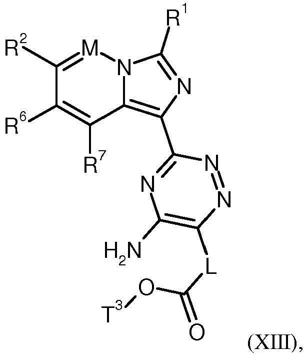 in welcher M, L, R¹, R², R⁶, R⁷ und T³ jeweils die oben angegebenen Bedeutungen haben,
   umsetzt und schliesslich in einem inerten Lösungsmittel gegebenenfalls in Gegenwart einer geeigneten Base zu einer Verbindung der Formel (I-D) 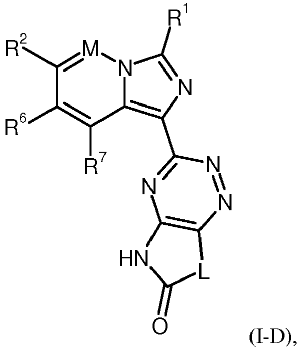 in welcher M, L, R¹, R², R⁶ und R⁷ jeweils die oben angegebenen Bedeutungen haben,, cyclisiert,
gegebenenfalls die resultierenden Verbindungen der Formel (I-A), (I-B), (I-C) und (I-D) mit den entsprechenden (i) Lösungsmitteln und/oder (ii) Säuren oder Basen in ihre Solvate, Salze und/oder Solvate der Salze überführt.

Die Verbindungen der Formeln (I-A), (I-B), (I-C) und (I-D) bilden zusammen die Gruppe der erfindungsgemäßen Verbindungen der Formel (I).

Inerte Lösungsmittel für den Verfahrensschritt (II) + (III) → (IV) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol oder Methanol.

Geeignete Basen für den Verfahrensschritt (II) + (III) → (IV) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat oder Natriummethanolat.

Die Reaktion (II) + (III) → (IV) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (IV) → (I-A) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (IV) → (I-A) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (IV) → (I-A) eignen sich beispielsweise Diiodmethan, eine Mischung aus Cäsiumiodid, Iod und Kupfer-(I)-iodid oder Kupfer -(II)-bromid.

Der Verfahrensschritt (IV) → (I-A) erfolgt im Fall von Diiodmethan als Halogenquelle mit einem Molverhältnis von 10 bis 30 Mol Isopentylnitrit und 10 bis 30 Mol des Iod-Äquivalents bezogen auf 1 Mol der Verbindung der Formel (IV).

Inerte Lösungsmittel für den Verfahrensschritt (I-A) → (I-B) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), *N,N'*-Dimethylpropylenharnstoff (DMPU), *N*-Methylpyrrolidon (NMP), Pyridin oder auch Wasser. Ebenso ist es möglich, Gemische der genannten Lösungsmittel einzusetzen. Bevorzugt ist DMF.

Die Reduktion (I-A) → (I-B) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (I-A) → (I-B) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (II) + (V) → (VI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Geeignete Basen für den Verfahrensschritt (II) + (V) → (VI) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Natriummethanolat oder Natriumethanolat.

Die Reaktion (II) + (V) → (VI) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzungen (VI) → (VII) und (XI) → (XII) können in einem unter den Reaktionsbedingungen inerten Lösungsmittel oder ohne Lösungsmittel erfolgen. Bevorzugtes Lösungsmittel ist Sulfolan.

Die Reaktionen (VI) → (VII) und (XI) → (XII) erfolgen im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (XI) → (XII) ohne Lösungsmittel in einem Temperaturbereich von 0°C bis +50°C bei Normaldruck.

Der Verfahrensschritt (VII) → (VIII) erfolgt durch Reaktion mit Natriumazid unter intermediärer Bildung der Azid-Derivate, die direkt weiter zu den entsprechenden Aminen reduziert werden. Inerte Lösungsmittel für die Azidbildung sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin, Acetonitril oder Sulfolan. Ebenso ist es möglich, Gemische der genannten Lösungsmittel einzusetzen. Bevorzugt ist DMF.

Die Azidbildung erfolgt erfolgt im Allgemeinen in einem Temperaturbereich von +50°C bis +100°C, bevorzugt von +60°C bis +80°C, bei Normaldruck.

Die Reduktion erfolgt in einem inerten Lösungsmittel wie beispielsweise Alkoholen wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion erfolgt bei +10°C bis +30°C mit Wasserstoff in Verbindung mit Übergangsmetallkatalysatoren wie beispielsweise Palladium (10% auf Aktivkohle), Platindioxid oder Palladiumhydroxid, oder ohne Wasserstoff mit Zinn(II)chlorid und Salzsäure.

Alternativ kann die Umsetzung (VII) → (VIII) auch in einer Stufe analog zum Verfahrensschritt (XII) → (XIII) durchgeführt werden.

Die Cyclisierungen (VIII) → (I-C) und (XIII) → (I-D) erfolgen in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für die Verfahrensschritte (VIII) → (I-C) und (XIII) → (I-D) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]-non-5-en (DBN). Bevorzugt ist Kalium-tert.-butylat.

Die Reaktionen (VIII) → (I-C) und (XIII) → (I-D) erfolgen im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgt die Cyclisierung zu (I-C) bzw. (I-D) direkt bei der Reduktion des Azids zum entsprechenden Amin (VIII) bzw. bei der Umsetzung (XII) → (XIII) ohne Zugabe weiterer Reagentien.

In einer alternativen Durchführung der Verfahren [C] bzw. [D] werden die Umwandlungen (VII) → (VIII) →(I-C) bzw. (IX) + (X) → (XI) → (XII) → (XIII) → (I-D) ohne Isolierung der Zwischenstufen durchgeführt.

Inerte Lösungsmittel für den Verfahrensschritt (II) → (IX) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Ethanol.

Geeignete Basen für den Verfahrensschritt (II) → (IX) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt Triethylamin.

Die Reaktion (II) → (IX) erfolgt im Allgemeinen in einem Temperaturbereich von 0°C bis +60°C, bevorzugt von +10°C bis +30°C. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (IX) + (X) → (XI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (IX) + (X) → (XI) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzung (XI) → (XII) kann in einem unter den Reaktionsbedingungen inerten Lösungsmittel oder ohne Lösungsmittel erfolgen. Bevorzugtes Lösungsmittel ist Sulfolan.

Die Reaktion (XI) → (XII) erfolgt im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (XI) → (XII) ohne Lösungsmittel in einem Temperaturbereich von 0°C bis +50°C bei Normaldruck.

Der Verfahrensschritt (XII) → (XIII) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N,N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (XII) → (XIII) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Cyclisierung (XIII) → (I-D) erfolgt in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), N-Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für den Verfahrensschritt (XIII) → (I-D) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (XIII) → (I-D) erfolgt im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgen die Umsetzungen (XI) → (XII) → (XIII) → (I-D) simultan in einer Eintopfreaktion ohne Isolierung der Zwischenstufen.

Die beschriebenen Herstellverfahren können durch die folgenden Syntheseschemata (Schema 1 bis 3) beispielhaft verdeutlicht werden: 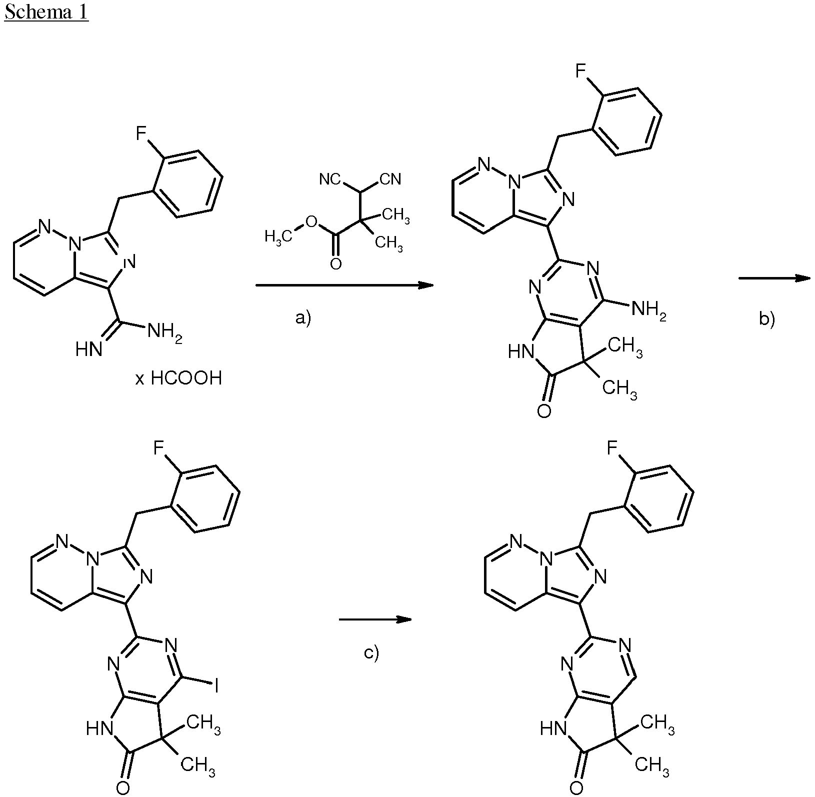 [a): Kalium-tert.-butylat, tert.-Butanol, Rückfluss; b]: Isopentylnitrit, Diiodmethan, 85°C; c): Palladium auf Kohle (10%-ig), Wasserstoff, DMF]. 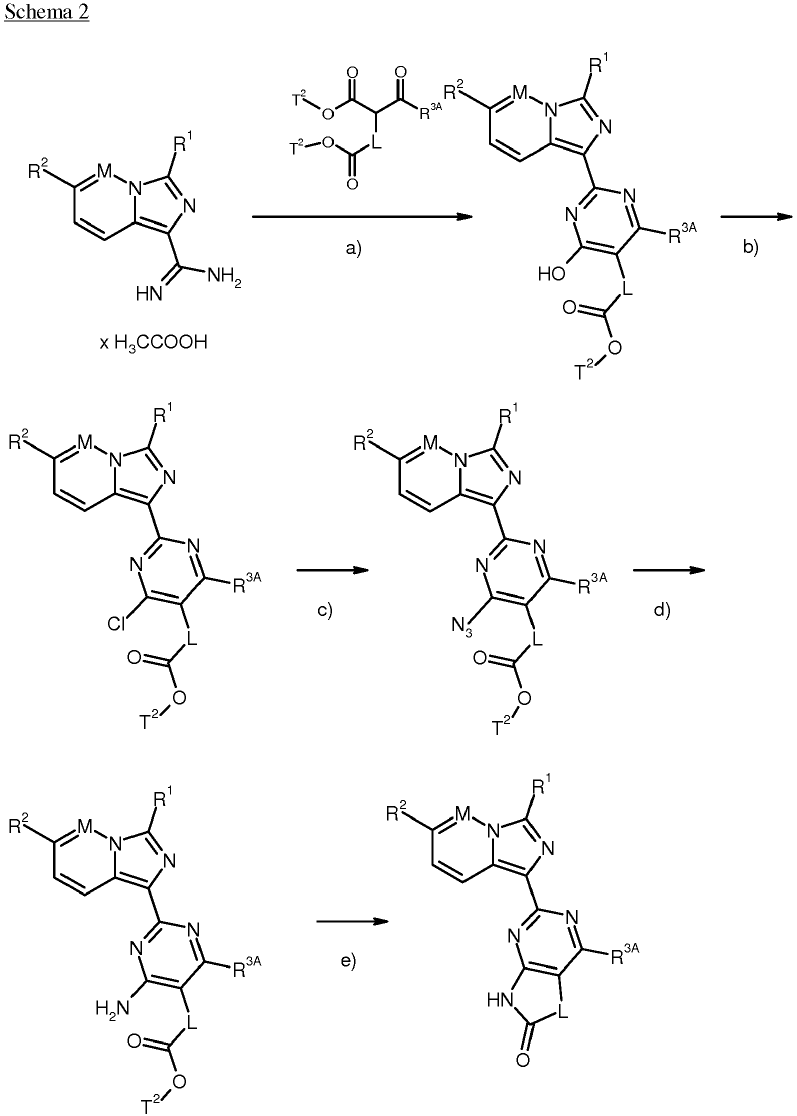 [a): NaOMe, MeOH; b): POCl₃; Sulfolan; c): NaN₃, DMF; d): Pd/C, H₂, DMF; e) KOt-Bu, THF]. 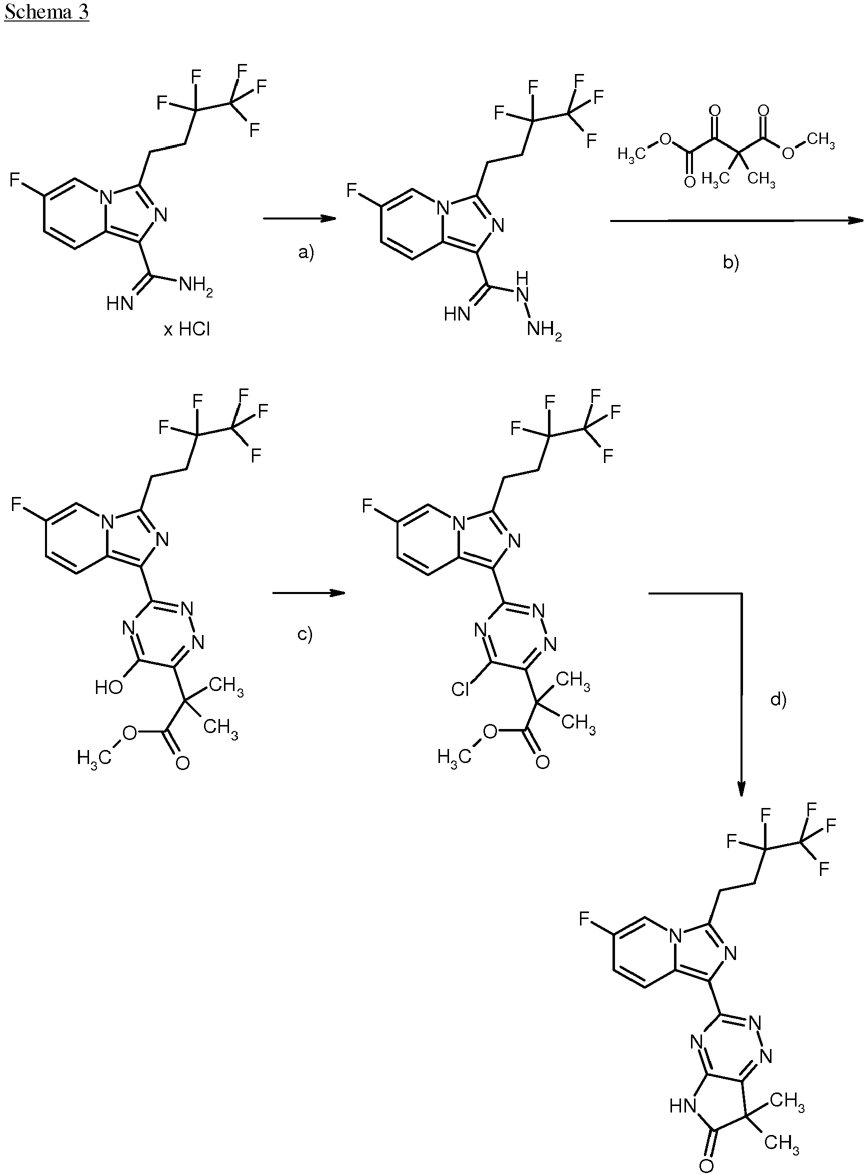 [a): Hydrazinhydrat, NEt₃, EtOH b): EtOH c): POCl₃; d): konz. NH₃].

Die Verbindungen der Formeln (III), (V) und (X) sind kommerziell erhältlich, literaturbekannt oder können in Analogie zu literaturbekannten Verfahren hergestellt werden.

Die Verbindungen der Formel (II) sind literaturbekannt (siehe z.B. WO 2010/065275) oder können hergestellt werden, indem man eine Verbindung der Formel (XIV) 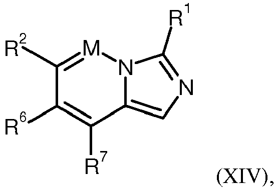 in welcher M, R¹, R², R⁶ und R⁷ jeweils die oben angegebenen Bedeutungen haben,
in einem inerten Lösungsmittel mit einem geeigneten Halogenieriungsmittel in eine Verbindung der Formel (XV) 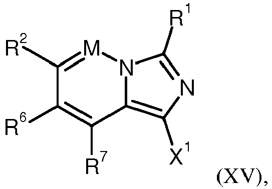
in welcher M, R¹, R², R⁶ und R⁷ jeweils die oben angegebenen Bedeutungen haben und
X¹ für Halogen, insbesondere Brom oder Iod, steht,
überführt, diese anschliessend in einem inerten Lösungsmittel zu einer Verbindung der Formel (XVI) 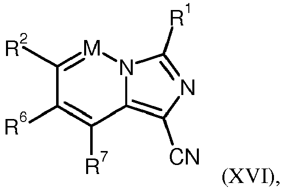 in welcher M, R¹, R², R⁶ und R⁷ jeweils die oben angegebenen Bedeutungen haben,
umsetzt, diese im Folgenden in einem inerten Lösungsmittel in eine Verbindung der Formel (II) 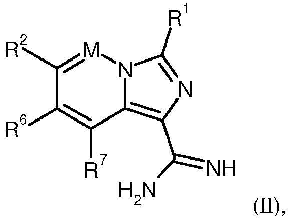 in welcher M, R¹, R², R⁶ und R⁷ jeweils die oben angegebenen Bedeutungen haben, überführt.

Als inerte Lösungsmittel für die Halogenierung (XIV) → (XV) eignen sich beispielsweise Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Dichlormethan.

Als Bromierungsmittel im Verfahrensschritt (XIV) → (XV) eignet sich elementares Brom mit Essigsäure, 1,3-Dibrom-5,5-dimethylhydantoin sowie insbesondere *N*-Bromsuccinimid (NBS).

Als Iodierungsmittel im Verfahrensschritt (XIV) → (XV) eignet sich insbesondere N-Iodsuccinimid (NIS).

Die Halogenierung (XIV) → (XV) wird im Allgemeinen in einem Temperaturbereich von -10°C bis +50°C durchgeführt, vorzugweise zwischen 0°C und +30°C. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XV) → (XVI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethyl-propylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (XV) → (XVI) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (XVI) → (II) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in Essigsäure unter Bildung des Amidins (VII) bei +50 bis +150°C.

Das beschriebene Herstellverfahren kann durch das folgende Syntheseschema (Schema 4) beispielhaft verdeutlicht werden: 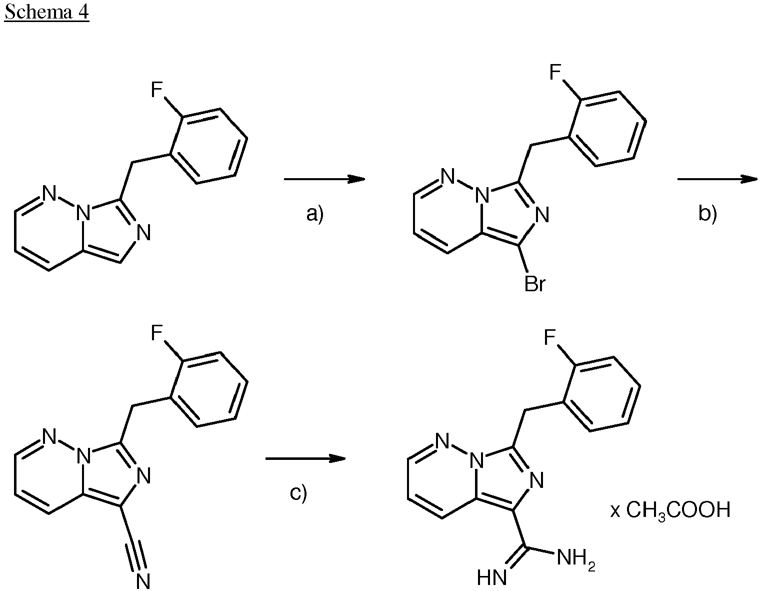 [a): N-Bromsuccinimid, Dichlormethan; b): Kupfer-(I)-cyanid, DMSO, 170°C; c): 1. Natriummethanolat, Methanol, 2. NH₄Cl, Essigsäure, Rückfluss].

Die Verbindungen der Formel (XIV) können hergestellt werden, indem man eine Verbindung der Formel (XVII) 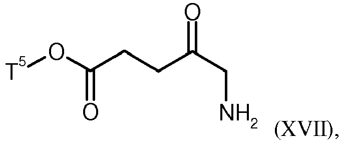 in welcher
T⁵ für (C₁-C₄)-Alkyl steht,
in einem inerten Lösungsmittel in Gegenwart einer geeigneten Base mit Hydrazin-Hydrat zu der Verbindung der Formel (XVIII) 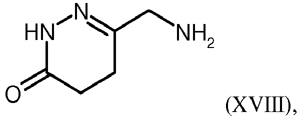 cyclisiert, die anschliessend in einem inerten Lösungsmittel in Gegenwart einer geeigneten Base mit einer Verbindung der Formel (XIX) 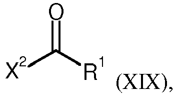
in welcher R¹ die oben angegebene Bedeutung hat und
X² für Halogen, insbsondere Chlor oder Brom, steht,
zu einer Verbindung der Formel (XX) 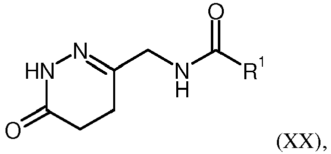 in welcher R¹ die oben angegebene Bedeutung hat,
umsetzt, diese dann zu einer Verbindung der Formel (XXI) 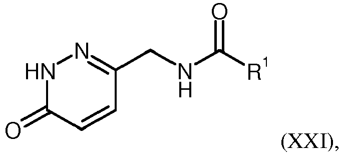 in welcher R¹ die oben angegebene Bedeutung hat,
oxidiert, diese weiterhin ohne Lösungsmittel oder in einem inerten Lösungsmittel mit Phosphoroxychlorid zu einer Verbindung der Formel (XXII) 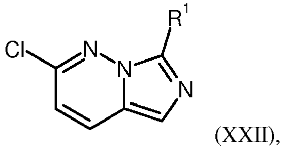 in welcher R¹ die oben angegebene Bedeutung hat,
cyclisiert, und abschließend in einem inerten Lösungsmittel in Gegenwart einer geeigneten Base hydriert.

Die Verbindungen der Formel (XVII) und (XVIII) sind kommerziell erhältlich, literaturbekannt oder können in Analogie zu literaturbekannten Verfahren hergestellt werden.

Inerte Lösungsmittel für den Verfahrensschritt (XVII) → (XVIII) sind Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Ethanol.

Geeignete Basen für den Verfahrensschritt (XVII) → (XVIII) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (XVII) → (XVIII) wird im Allgemeinen in einem Temperaturbereich von +20°C bis +150°C, bevorzugt bei +80°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XVIII) + (XIX) → (XX) sind Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, Halogenkohlenwasserstoffe wie Dichlormethan, Trichlormethan, Tetrachlormethan, Trichlorethylen oder Chlorbenzol, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Acetonitril.

Geeignete Basen für den Verfahrensschritt (XVIII) + (XIX) → (XX) sind Alkalicarbonate wie Lithium-, Natrium-, Kalium- oder Cäsiumcarbonat, oder organische Amine wie Triethylamin, Diisopropylethylamin, Pyridin, 1,8-Diazabicyclo[5.4.0]undec-7-en (DBU) oder 1,5-Diazabicyclo-[4.3.0]non-5-en (DBN). Bevorzugt ist Triethylamin.

Die Reaktion (XVIII) + (XIX) → (XX) wird im Allgemeinen in einem Temperaturbereich von - 20°C bis +40°C, bevorzugt bei 0°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Oxdiation (XX) → (XXI) erfolgt bevorzugt in einer organischen Säure wie beispielsweise Ameisensäure oder Essigsäure, in Gegenwart von elementarem Brom bei einer Temperatur von +40°C bis +100°C.

Die Cyclisierung (XXI) → (XXII) erfolgt ohne Lösungsmittel oder in einem Lösungsmittel, welches unter den Reaktionsbedingungen inert ist. Geeignete Lösungsmittel sind beispielsweise Kohlenwasserstoffe wie Benzol, Xylol, Toluol, Hexan, Cyclohexan oder Erdölfraktionen oder andere Lösungsmittel wie Dimethylformamid (DMF), Dimethylsulfoxid (DMSO), *N,N'*-Dimethyl-propylenharnstoff (DMPU), *N*-Methylpyrrolidon (NMP), Pyridin, Sulfolan oder Acetonitril. Bervorzugt wird Sulfolan eingesetzt.

Die Cyclisierung (XXI) → (XXII) wird im Allgemeinen in einem Temperaturbereich von +50°C bis +140°C, bevorzugt bei +80°C bis +120°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (XXII) → (XIV) erfolgt in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Essigsäureethylester, Dimethylformamid (DMF), Dimethylsulfoxid (DMSO), *N,N'*-Dimethylpropylenharnstoff (DMPU), N-Methylpyrrolidon (NMP), Pyridin, Sulfolan oder Acetonitril. Bevorzugt ist Essigsäureethylester.

Geeignete Basen für den Verfahrensschritt (XXII) → (XIV) sind organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Cyclisierung (XXII) → (XIV) wird im Allgemeinen in einem Temperaturbereich von 0°C bis +60°C, bevorzugt bei +10°C bis +30°C durchgeführt.

Das nachfolgende Schema (Schema 5) zeigt bespielhaft das zuvor beschriebene Herstellverfahren: 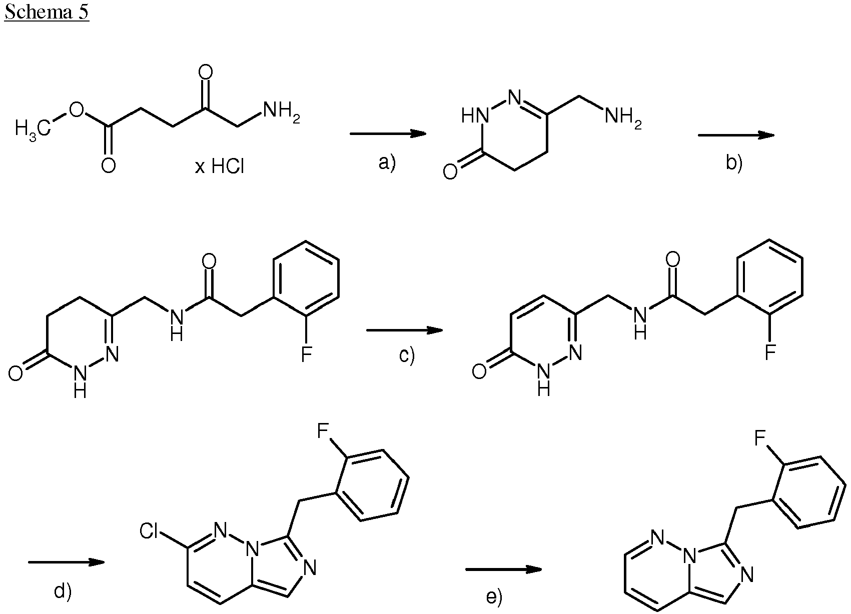 [a): Hydrazinhydrat, Triethylamin, Ethanol, Rückfluss; b): (2-Fluorphenyl)acetylchlorid, Triethylamin, Acetonitril; c): Brom, Essigsäure, 50°C; d): Phosphoroxychlorid, Sulfolan, 100°C; e): Palladium auf Kohle (5%), Triethylamin, Wasserstoff, Essigsäureethylester].

Die erfindungsgemäßen Verbindungen wirken als potente Stimulatoren der löslichen Guanylatcyclase und weisen ein gleiches oder verbessertes therapeutisches Profil gegenüber den aus dem Stand der Technik bekannten Verbindungen auf, wie beispielsweise hinsichtlich ihrer *in-vivo* Eigenschaften, wie beispielsweise ihrem pharmakokinetischem und pharmakodynamischem Verhalten und/oder ihrer Dosis-Wirkungsbeziehung und/oder ihres Sicherheitsprofils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-hihibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

### Methode 1 (LC-MS):

Instrument: Waters ACQUITY SQD UPLC System; Säule: Waters Acquity UPLC HSS T3 1,8µ 50 x 1 ml; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Methode 2 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 3 (LC-MS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Ausgangsverbindungen und Intermediate:

### Beispiel 1A

### 2-(2-Fluorphenyl)-N-[(6-oxo-1,4,5,6-tetrahydropyridazin-3-yl)methyl]acetamid

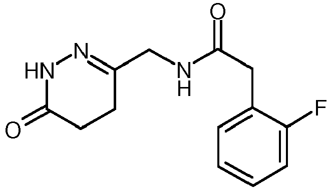

200.00 g (1.101 mol) Methyl-5-amino-4-oxopentanoat-Hydrochlorid wurden in Ethanol (3500 ml) vorgelegt, mit 64.28 ml (1.321 mol) Hydrazinhydrat versetzt und anschliessend 45 min zum Rückfluss erhitzt. Nach Abkühlen wurde Triethylamin (152 ml) zugegeben und das Gemisch bis zur Trockene eingeengt. Zum Rückstand wurde Wasser (500 ml) gegeben und eingeengt. Danach wurde Ethanol (500 ml) zugegeben, eingeengt, danach zweimal Toluol (500 ml) zugegeben und bis zur Trockene eingeengt. Der Rückstand (140 g) wurde in Acetonitril (500 ml) gelöst und langsam bei 0°C zu einer Lösung von 307.85 g (1.784 mol) (2-Fluorphenyl)acetylchlorid (Herstellung: Journal of Organic Chemistry; 22; 1957; 879) und 304.86 ml (2.202 mol) Triethylamin in Acetonitril (1500 ml) und Molsieb getropft. Es wurde 3 Tage bei 20°C gerührt. Danach wurde filtriert und der Niederschlag wurde mit tert.-Butylmethylether gewaschen und anschliessend getrocknet. Es wurden 458 g der Zielverbindung erhalten (90 % d. Th.).

LC-MS (Methode 1): Rt = 0.57 min; MS (EIpos): m/z = 264 [M+H]⁺.

### Beispiel 2A

### 2-(2-Fluorphenyl)-N-[(6-oxo-1,6-dihydropyridazin-3-yl)methyl]acetamid

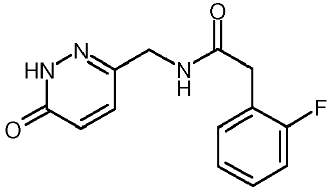

458 g (1.740 mol) der unter Beispiel 1A erhaltenen Verbindung wurden in Essigsäure (2250 ml) vorgelegt und auf 50°C erwärmt. Bei dieser Temperatur wurde unter starkem Rühren 98.16 ml (1.914 mol) Brom zugetropft und anschliessend 3h bei 50°C weitergerührt. Nach Abkühlen wurde das Reaktionsgemisch bis zur Trockene eingeengt. Der Rückstand wurde mit gesättigter wässriger Natriumhydrogencarbonatlösung (4800 ml) verrührt. Danach wurde filtriert und der Niederschlag wurde mit wenig Wasser gewaschen. Das Filtrat wurde zweimal mit Essigsäureethylester extrahiert. Die organischen Phasen wurden vereinigt, getrocknet und eingeengt. Es wurden 117 g der Zielverbindung erhalten (25 % d. Th.).

LC-MS (Methode 1): Rt = 0.56 min; MS (EIpos): m/z = 262 [M+H]⁺.

¹H-NMR (400 MHz, DMSO-d₆): δ [ppm] = 3.54 (s, 2H), 4.16 (d, 2H), 6.86 (d, 1H), 7.12-7.16 (m, 2H), 7.27-7.35 (m, 3H), 8.62 (t, 1H), 12.88 (s, 1H).

### Beispiel 3A

### 2-Chlor-7-(2-fluorbenzyl)imidazo[1,5-b]pyridazin

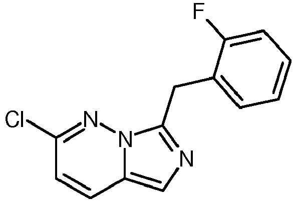

65.00 g (248.79 mmol) der unter Beispiel 2A erhaltenen Verbindung wurden in Sulfolan (780 ml) vorgelegt, mit 185.52 ml (1.990 mol) Phosphoroxychlorid versetzt und anschliessend 3h bei 100°C gerührt. Danach wurde am Hochvakuum der Überschuss an Phosphoroxychlorid abdestilliert, der Rückstand in Essigsäureethylester aufgenommen und zu einer gesättigten wässrigen Natriumhydrogencarbonatlösung gegeben. Es wurde mit Wasser verdünnt und danach mit Essigsäureethylester extrahiert. Die organischen Phasen wurden vereinigt, mit Wasser gewaschen, über Natriumsulfat getrocknet und eingeengt. Der Rückstand wurde mittels Chromatographie an Kieselgel (Laufmittel: Dichlormethan/Methanol 20:1 → 5:1 (v/v)) gereinigt, anschliessend mit Wasser gewaschen und mittels Chromatographie an Kieselgel (Laufmittel: Dichlormethan/Methanol 100:1 v/v) gereinigt. Es wurden 23.6 g der Zielverbindung erhalten (36 % d. Th.).

LC-MS (Methode 1): Rt = 1.00 min; MS (EIpos): m/z = 262 [M+H]⁺.

¹H-NMR (400 MHz, DMSO-d₆): δ [ppm] = 4.40 (s, 2H), 6.84 (d, 1H), 7.10-7.33 (m, 4H), 7.55 (s, 1H), 8.19 (d, 1H).

### Beispiel 4A

### 7-(2-Fluorbenzyl)imidazo[1,5-b]pyridazin

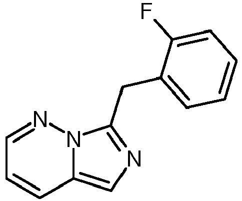

2.004 g Palladium auf Kohle (5%-ig) wurden unter Argon vorgelegt und anschliessend mit 20.04 g (76.58 mmol) der unter Beispiel 3A erhaltenen Verbindung in Essigsäureethylester (750 ml) versetzt. Danach wurde 21.348 ml (153.159 mmol) Triethylamin zugegeben und das Reaktionsgemisch wurde 16 Stunden bei Wasserstoff-Normaldruck und 20°C hydriert. Anschliessend wurde das Reaktionsgemisch abermals mit der oben angegebenen Menge an Katalysator versetzt und eine weitere Nacht bei Wasserstoff-Normaldruck und 20°C hydriert. Im Anschluss daran wurde über Celite filtriert, mit Ethanol nachgewaschen, eingeengt und am Hochvakuum getrocknet. Es wurden 22.79 g der Zielverbindung erhalten (ca. 100 % d. Th., verunreinigt mit Triethylamin).

LC-MS (Methode 1): Rt = 0.77 min; MS (EIpos): m/z = 228 [M+H]⁺.

¹H-NMR (400 MHz, DMSO-d₆): δ [ppm] = 4.44 (s, 2H), 6.70 (dd, 1H), 7.08-7.31 (m, 4H), 7.45 (s, 1H), 8.09 (dd, 1H), 8.28 (dd, 1H).

### Beispiel 5A

### 5-Brom-7-(2-fluorbenzyl)imidazo[1,5-b]pyridazin

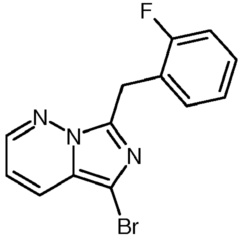

22.46 g (98.837 mmol) der unter Beispiel 4A erhaltenen Verbindung wurden in Dichlormethan (400 ml) vorgelegt und mit 17.591 g (98.837 mmol) N-Bromsuccinimid versetzt. Danach wurde das Gemisch 10 min bei 20°C gerührt. Anschliessend wurde das Reaktionsgemisch mit Wasser versetzt, die Phasen wurden separiert und die organische Phase wurde mit Wasser gewaschen. Die wässrige Phase wurde zweimal mit Dichlormethan extrahiert, die vereinigten organischen Phasen wurden mit gesättigter wässriger Natriumchloridlösung gewaschen, über Natriumsulfat getrocknet, filtriert und eingeengt. Es wurden 22.78 g der Zielverbindung erhalten (75% d. Th.).

LC-MS (Methode 1): Rt = 1.05 min; MS (EIpos): m/z = 306, 308 [M+H, Brom-Muster]⁺.

¹H-NMR (400 MHz, DMSO-d₆): δ [ppm] = 4.45 (s, 2H), 6.81 (dd, 1H), 7.12-7.34 (m, 4H), 7.94 (dd, 1H), 8.28 (dd, 1H).

### Beispiel 6A

### 7-(2-Fluorbenzyl)imidazo[1,5-b]pyridazin-5-carbonitril

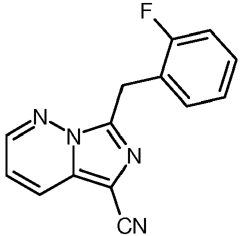

1.00 g (3.266 mmol) der unter Beispiel 5A erhaltenen Verbindung wurden in trockenem DMSO (25 ml) vorgelegt, mit 1.170 g (13.066 mmol) Kupfer-(I)-cyanid versetzt und 3.5h unter Rühren auf 170°C erhitzt. Danach wurde über Celite filtriert und mit Essigsäureethylester und Tetrahydrofuran nachgewaschen. Anschliessend wurde das Filtrat viermal mit einer Mischung aus gesättiger wässriger Ammoniumchlorid-Lösung/Ammoniakwasser (33 %) (3:1, v/v) extrahiert und einmal mit gesättigter wässriger Natriumchlorid-Lösung gewaschen. Die Phasen wurden separiert und die organische Phase wurde mit Natriumsulfat getrocknet, filtriert und eingeengt. Der Rückstand wurde im Ultraschallbad mit Ethanol aufgeschlossen und danach mit Wasser versetzt. Der sich bildende Niederschlag wurde abfiltriert, mit Ethanol nachgewaschen und anschliessend am Hochvakuum getrocknet. Es wurden 586 mg der Zielverbindung erhalten (71% d. Th.).

LC-MS (Methode 1): Rt = 0.95 min; MS (EIpos): m/z = 253 [M+H]⁺.

¹H-NMR (400 MHz, DMSO-d₆): δ [ppm] = 4.49 (s, 2H), 7.13-7.35 (m, 5H), 8.40 (d, 1H), 8.61 (d, 1H).

### Beispiel 7A

### 7-(2-Fluorbenzyl)imidazo[1,5-b]pyridazin-5-carboximidamid-Acetat

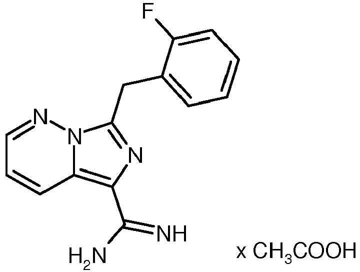

Zu 125 mg (2.315 mmol) Natriummethanolat in Methanol (10 ml) wurden 584 mg (2.315 mmol) der unter Beispiel 6A hergestellten Verbindung gegeben und 16 Stunden bei 20°C gerührt. Danach wurden 148 mg (2.778 mmol) Ammoniumchlorid und Essigsäure (0.517 ml) zugegeben und für 8h zum Rückfluss erhitzt. Danach wurde das Reaktionsgemisch bis zur Trockene eingeengt, der Rückstand in Wasser und Essigsäureethylester aufgenommen und mit 1N Natronlauge versetzt. Die Phasen wurden getrennt und die wässrige Phase wurde zweimal mit Essigsäureethylester extrahiert. Die vereinigten organischen Phasen wurden mit Natriumsulfat getrocknet, filtriert, eingeengt und anschliessend im Hochvakuum getrocknet. Es wurden 543 mg der Zielverbindung erhalten (71% d. Th.).

LC-MS (Methode 1): Rt = 0.63 min; MS (EIpos): m/z = 270 [M+H]⁺.

### Beispiel 8A

### Methyl-3,3-dicyan-2,2-dimethylpropanoat

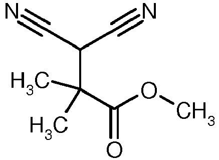

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben und es wurde 16 Stunden bei 20°C gerührt. Danach wurden nochmals 5.876 ml (45.411 mmol) Methyl-2-brom-2-methylpropanoat zugegeben und es wurde 16 Stunden auf 50°C erhitzt. Dann wurden abermals 1.762 ml (13.623) mmol) Methyl-2-brom-2-methylpropanoat zugegeben und es wurde weitere 4h auf 50°C erhitzt. Das Reaktionsgemisch wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, mit Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.

Ausbeute: 6.47 g (85% d. Th.)

1H-NMR (400 MHz, DMSO-d6): δ [ppm] = 1.40 (s, 6H), 3.74 (s, 3H), 5.27 (s, 1H).

### Beispiel 9A

### 4-Amino-2-[7-(2-fluorbenzyl)imidazo[1,5-b]pyridazin-5-yl]-5,5-dimethyl-5,7-dihydro-6H-pyrrolo[2,3-d]pyrimidin-6-on

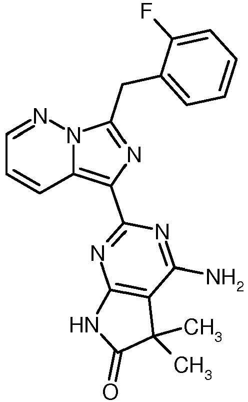

543 mg (1.649 mmol) Beispiel 7A wurden in tert.-Butanol (30 ml) vorgelegt und mit 222 mg (1.979 mmol) Kalium-tert.-butylat versetzt. Anschliessend wurden 301 mg (1.979 mmol) Beispiel 8A in tert.-Butanol (20 ml) zugetropft und die Mischung 2 Stunden zum Rückfluss erhitzt. Nach Abkühlen wurde mit Wasser versetzt und dreimal mit Essigsäureethylester extrahiert. Die vereinigten organischen Phasen wurden eingeengt und der Rückstand mit wenig Essigsäureethylester und einigen Tropfen Ethanol versetzt. Anschliessend wurde Diethylether zugegeben. Es bildete sich ein Niederschlag, der abfiltriert wurde. Nach Trocknung am Hochvakuum wurden 290 mg der Zielverbindung erhalten (43 % d. Th.).

LC-MS (Methode 1): Rt = 0.85 min; MS (EIpos): m/z = 404 [M+H]⁺.

¹H-NMR (400 MHz, DMSO-d₆): δ [ppm] = 1.32 (s, 6H), 4.49 (s, 2H), 6.66 (s br, 2H), 6.95 (dd, 1H), 7.12-7.22 (m, 2H), 7.24-7.33 (m, 2H), 8.41 (dd, 1H), 8.98 (dd, 1H), 10.86 (s br, 1H).

### Beispiel 10A

### 7-(2-Fluorbenzyl)imidazo[1,5-b]pyridazin-5-carboximidohydrazid

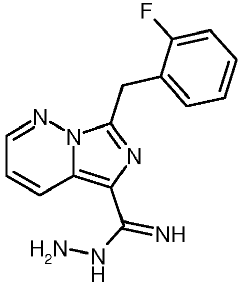

1.600 g (4.858 mmol) Beispiel 7A wurden in 40 ml Ethanol gelöst und bei 0°C mit 1.966 g (19.433 mmol) Triethylamin sowie 304 mg (4.858 mmol) Hydrazinhydrat (80%-ige Lösung in Wasser) versetzt. Das Gemisch wurde über Nacht bei RT gerührt und anschließend eingeengt. Der Rückstand wurde ohne weitere Reinigung in der nächsten Stufe eingesetzt. Es wurden 1.63 g (73 % d. Th., 62 %ige Reinheit) der Titelverbindung erhalten.

LC-MS (Methode 2): Rₜ = 1.43 min; MS (ESIpos): m/z = 285 (M+H)⁺

### Beispiel 11A

### Methyl-2-{3-[7-(2-fluorbenzyl)imidazo[1,5-b]pyridazin-5-yl]-5-hydroxy-1,2,4-triazin-6-yl}-2-methylpropanoat

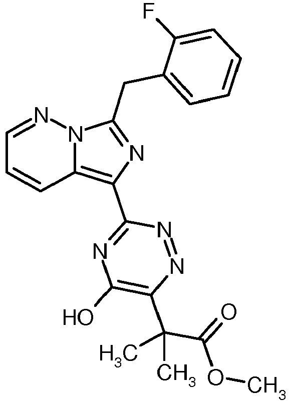

1.010 g (5.366 mmol) Dimethyl-2,2-dimethyl-3-oxobutandioat (beschrieben in J. Am. Chem. Soc. 124(14), 3680-3691; 2002) wurden in 30 ml Ethanol vorgelegt und zum Rückfluss erhitzt. Dazu wurden 1.630 g (ca. 3.577 mmol) der unter Beispiel 10A erhaltenen Verbindung als Suspension in 30 ml Ethanol langsam zugetropft. Das Gemisch wurde über Nacht zum Rückfluß erhitzt. Nach Einengen wurde der Rückstand ohne weitere Reinigung in der nächsten Stufe eingesetzt. Es wurden 2.00 g (40 % d. Th., 30 %ige Reinheit) der Titelverbindung erhalten.

LC-MS (Methode 1): Rₜ = 1.01 min; MS (ESIpos): m/z = 423 (M+H)⁺

### Beispiel 12A

### 6-Chlor-1-iod-3-(2,3,6-trifluorbenzyl)imidazo[1,5-a]pyridin

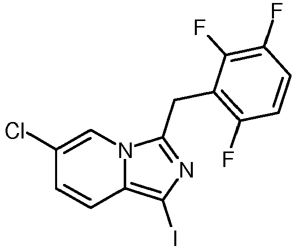

Die Titelverbindung wurde in Analogie zur Vorschrift in WO 2010/065275, Beispiel 58, Seite 46-48 hergestellt.

LC-MS (Methode 1): Rₜ = 1.24 min; MS (ESIpos): m/z = 423 (M+H)⁺

### Beispiel 13A

### 6-Chlor-3-(2,3,6-trifluorbenzyl)imidazo[1,5-a]pyridin-1-carbonitril

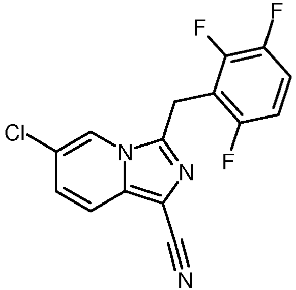

10.00 g (23.664 mmol) Beispiel 12A und 2.331 g (26.031 mmol) Kupfer-(I)-cyanid wurden in DMSO 5 h bei 150°C gerührt. Der Ansatz wurde über Celite filtriert, mit Essigsäureethylester nachgewaschen und das Filtrat anschliessend viermal mit einer 3:1 Mischung aus gesättigter wässriger Ammoniumchloridlösung und konz. Ammoniakwasser (33%) extrahiert. Danach wurde mit gesättigter wässriger Natriumchloridlösung gewaschen, die Phasen separiert und die organische Phase über Natriumsulfat getrocknet, filtriert und eingeengt. Der Rückstand wurde über Nacht am Hochvakuum getrocknet. Es wurden 7.30 g der Titelverbindung erhalten (95% d. Th.).

LC-MS (Methode 3): Rt = 1.09 min; MS (ESIpos): m/z = 322 (M+H)⁺

¹H-NMR (400 MHz, DMSO-d₆): δ [ppm] = 4.56 (s, 2H), 7.18-7.24 (m, 1H), 7.36 (d, 1H), 7.48-7.57 (m, 1H), 7.87 (d, 1H), 9.00 (s, 1H).

### Beispiel 14A

### 6-Chlor-3-(2,3,6-trifluorbenzyl)imidazo[1,5-a]pyridin-1-carboximidamid

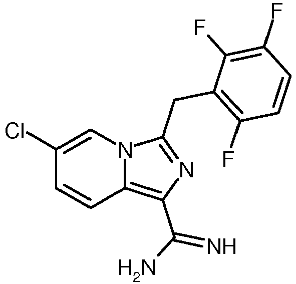

3.98 g (12.372 mmol) Beispiel 13A wurden in Analogie zur Vorschrift in WO 2010/065275, Beispiel 58, Step H, Seite 49 umgesetzt. Es wurden 3.54 g der Titelverbindung erhalten (84% d. Th.).

LC-MS (Methode 1): Rₜ = 0.66 min; MS (ESIpos): m/z = 339 (M+H)⁺

¹H-NMR (400 MHz, DMSO-d₆): δ [ppm] = 4.61 (s, 2H), 7.18-7.24 (m, 1H), 7.47-7.57 (m, 2H), 8.08 (d, 1H), 8.79 (br d, 3H), 9.03 (s, 1H).

### Beispiel 15A

4-Amino-2-[6-chlor-3-(2,3,6-trifluorbenzyl)imidazo[1,5-a]pyridin-1-yl]-5,5-dimethyl-5,7-dihydro-6H-pyrrolo[2,3-d]pyrimidin-6-on

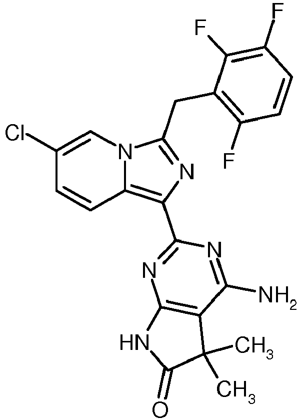

Die Verbindung ist beschrieben in WO 2010/065275, Beispiel 59, Seite 52. Es wurden 607 mg der Titelverbindung aus 500 mg Beispiel 14A erhalten (86% d. Th.).

LC-MS (Methode 2): Rt = 1.95 min; MS (ESIpos): m/z = 473 (M+H)⁺

### Beispiel 16A

### 6-Fluor-1-iod-3-(3,3,4,4,4-pentafluorbutyl)imidazo[1,5-a]pyridin

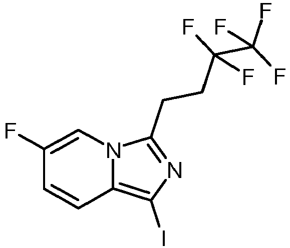

Die Titelverbindung wurde in Analogie zur Vorschrift in Beispiel 12A hergestellt.

LC-MS (Methode 3): Rt = 1.24 min; MS (ESIpos): m/z = 409 (M+H)⁺

### Beispiel 17A

### 6-Fluor-3-(3,3,4,4,4-pentafluorbutyl)imidazo[1,5-a]pyridin-1-carbonitril

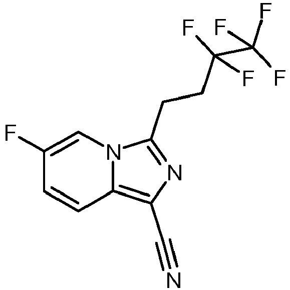

10.00 g (24.505 mmol) Beispiel 16A wurden in Analogie zur Vorschrift unter Beispiel 13A umgesetzt. Es wurden 6.98 g der Titelverbindung erhalten (92% d. Th.).

LC-MS (Methode 3): Rₜ = 1.07 min; MS (ESIpos): m/z = 308 (M+H)⁺

### Beispiel 18A

### 6-Fluor-3-(3,3,4,4,4-pentafluorbutyl)imidazo[1,5-a]pyridin-1-carboximidamid

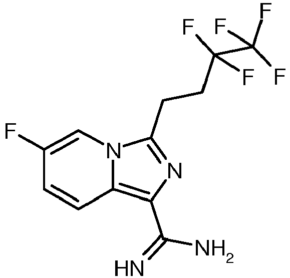

3.810 g (12.402 mmol) Beispiel 17A wurden in Analogie zur Vorschrift unter Beispiel 14A umgesetzt. Es wurden 2.95 g der Titelverbindung erhalten (73% d. Th.).

LC-MS (Methode 1): Rₜ = 0.64 min; MS (ESIpos): m/z = 325 (M+H)⁺

¹H-NMR (400 MHz, DMSO-d₆): δ [ppm] = 2.83-2.97 (m, 2H), 2H vermutlich unter Wassersignal, 7.53-7.58 (m, 1H), 8.16 (dd, 1H), 8.85 (s br, 3H), 8.92 (dd, 1H).

### Beispiel 19A

### 4-Amino-2-[6-fluor-3-(3,3,4,4,4-pentafluorbutyl)imidazo[1,5-a]pyridin-1-yl]-5,5-dimethyl-5,7-dihydro-6H-pyrrolo[2,3-d]pyrimidin-6-on

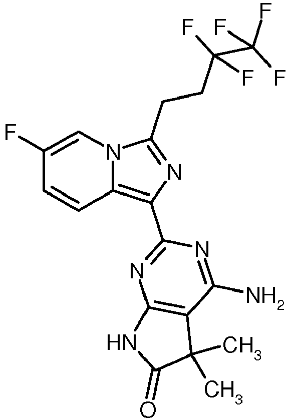

Die Verbindung ist beschrieben in WO 2010/065275, Beispiel 109, Seite 64. Sie wurde in Analogie zu den Vorschriften unter Beispiel 15A hergestellt.

LC-MS (Methode 2): Rₜ = 1.92 min; MS (ESIpos): m/z = 459 (M+H)⁺

### Beispiel 20A

### 6-Chlor-3-(2,3,6-trifluorbenzyl)imidazo[1,5-a]pyridin-1-carboximidohydrazid

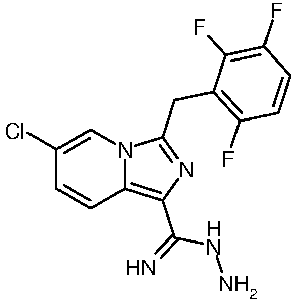

1.700 g (5.019 mmol) der Verbindung aus Beispiel 14A wurden in 35 ml Ethanol gelöst und bei 0°C mit 2.031 g (20.075 mmol) Triethylamin sowie 0.314 g (5.019 mmol) Hydrazinhydrat (80%-ige Lösung in Wasser) versetzt. Das Gemisch wurde über Nacht bei RT gerührt und anschließend am Rotationsverdampfer eingeengt. Es wurde eine Rohverbindung erhalten (2.08 g, 86 % d.Th., 73%ige Reinheit), die direkt weiter umgesetzt wurde.

LC-MS (Methode 3): Rt = 0.65 min; MS (ESIpos): m/z = 354 (M+H)⁺

### Beispiel 21A

### Methyl-2-{3-[6-chlor-3-(2,3,6-trifluorbenzyl)imidazo[1,5-a]pyridin-1-yl]-5-hydroxy-1,2,4-triazin-6-yl}-2-methylpropanoat

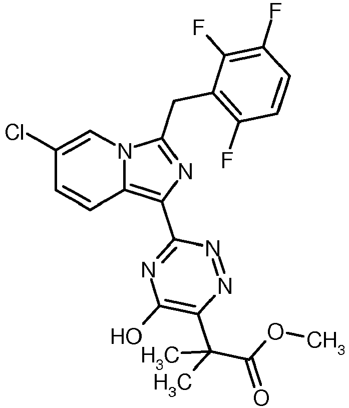

1.222 g (6.492 mmol) Dimethyl-2,2-dimethyl-3-oxobutandioat (beschrieben in J. Am. Chem. Soc. 124(14), 3680-3691; 2002) wurden in 30 ml Ethanol zum Rückfluss erhitzt. Danach wurde die Rohsubstanz aus Beispiel 20A (ca. 4.328 mmol) 20 ml Ethanol gelöst und zugetropft. Das Gemisch wurde über Nacht zum Rückfluss erhitzt. Nach dem Abkühlen wurde ein Feststoff abgesaugt, mit Ethanol gewaschen und das Filtrat eingeengt. Der Rückstand wurde für 30 min unter Rühren mit Diethylether versetzt und von einem sich bildenden Niederschlag filtriert und mit Diethylether nachgewaschen. Dieser Feststoff wurde über Nacht im Hochvakuum getrocknet. Es konnten so 1.67 g der Titelverbindung erhalten werden (55 % d. Th., 71%ige Reinheit).

LC-MS (Methode 1): Rₜ = 1.07 min; MS (ESIpos): m/z = 492 (M+H)⁺

### Beispiel 22A

### 6-Fluor-3-(3,3,4,4,4-pentafluorbutyl)imidazo[1,5-a]pyridin-1-carboximidohydrazid

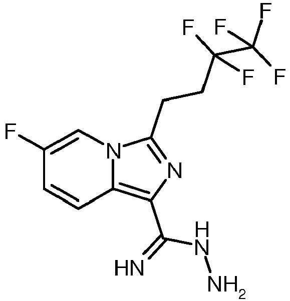

1.400 g (4.318 mmol) der Verbindung aus Beispiel 18A wurden in 30 ml Ethanol gelöst und bei 0°C mit 1.748 g (17.272 mmol) Triethylamin sowie 0.270 g (4.318 mmol) Hydrazinhydrat (80%-ige Lösung in Wasser) versetzt. Das Gemisch wurde über Nacht bei RT gerührt und anschließend am Rotationsverdampfer eingeengt. Es wurde eine Rohverbindung erhalten (1.70 g, 86 % d.Th., 74%ige Reinheit), die direkt weiter umgesetzt wurde.

LC-MS (Methode 3): Rt = 0.65 min; MS (ESIpos): m/z = 340 (M+H)⁺

### Beispiel 23A

### Methyl-2-{3-[6-fluor-3-(3,3,4,4,4-pentafluorbutyl)imidazo[1,5-a]pyridin-1-yl]-5-hydroxy-1,2,4-triazin-6-yl}-2-methylpropanoat

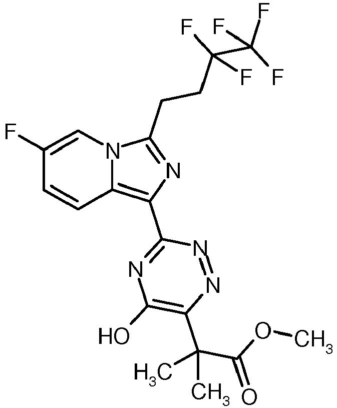

1.047 g (5.562 mmol) Dimethyl-2,2-dimethyl-3-oxobutandioat (beschrieben in J. Am. Chem. Soc. 124(14), 3680-3691; 2002) wurden in 20 ml Ethanol zum Rückfluss erhitzt. Danach wurde die Rohsubstanz aus Beispiel 22A (ca 3.708 mmol) in 20 ml Ethanol suspendiert und zugetropft. Das Gemisch wurde über Nacht zum Rückfluss erhitzt und nach dem Abkühlen eingeengt. Der Rückstand wurde für 30 min unter Rühren mit Diethylether versetzt, von einem sich bildenden Niederschlag filtriert und mit Diethylether nachgewaschen. Dieser Feststoff wurde über Nacht am Hochvakuum getrocknet. Es wurden 1.49 g der Titelverbindung erhalten (54 % d. Th., 64%ige Reinheit).

LC-MS (Methode 3): Rt = 1.10 min; MS (ESIpos): m/z = 478 (M+H)⁺

### Ausführungsbeispiele

### Beispiel 1

### 2-[7-(2-Fluorbenzyl)imidazo[1,5-b]pyridazin-5-yl]-4-iod-5,5-dimethyl-5,7-dihydro-6H-pyrrolo[2,3-d]pyrimidin-6-on

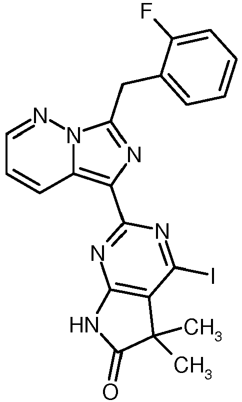

215 mg (0.535 mmol) Beispiel 9A wurden in Isopentylnitrit (1.548 ml) und Diiodmethan (4.045 ml) vorgelegt und für 3 Stunden auf 85°C erhitzt. Nach Abkühlen wurde das Reaktionsgemisch eingeengt und der Rückstand wurde mittels präparativer HPLC gereinigt (Acetonitril:Wasser (+0.05 % Ameisensäure) - Gradient). Es wurden 79 mg (28 % d. Th.) der Zielverbindung erhalten, sowie 20 mg von Beispiel 2.

LC-MS (Methode 1): Rₜ = 1.11 min; MS (EIpos): m/z = 515 [M+H]⁺.

¹H-NMR (400 MHz, DMSO-d₆): δ [ppm] = 1.40 (s, 6H), 4.52 (s, 2H), 6.95 (dd, 1H), 7.12-7.33 (m, 5H), 8.51 (dd, 1H), 8.72 (dd, 1H), 11.64 (s br, 1H).

### Beispiel 2

### 2-[7-(2-Fluorbenzyl)imidazo[1,5-b]pyridazin-5-yl]-4-hydroxy-5,5-dimethyl-5,7-dihydro-6H-pyrrolo[2,3-d]pyrimidin-6-on

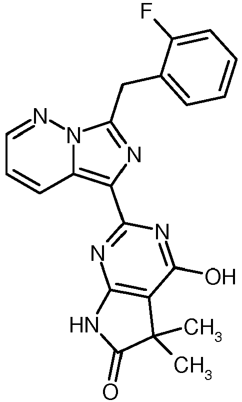

215 mg (0.535 mmol) Beispiel 9A wurden in Isopentylnitrit (1.548 ml) und Diiodmethan (4.045 ml) vorgelegt und für 3 Stunden auf 85°C erhitzt. Nach Abkühlen wurde das Reaktionsgemisch eingeengt und der Rückstand wurde mittels präparativer HPLC gereinigt (Acetonitril:Wasser (+0.05 % Ameisensäure) - Gradient). Es wurden 20 mg (9 % d. Th.) der Zielverbindung erhalten, sowie 79 mg von Beispiel 1.

LC-MS (Methode 1): Rₜ = 0.90 min; MS (EIpos): m/z = 405 [M+H]⁺.

¹H-NMR (400 MHz, DMSO-d₆): δ [ppm] = 1.31 (s, 6H), 4.55 (s, 2H), 7.12 (t, 1H), 7.18-7.23 (m, 2H), 7.30-7.36 (m, 2H), 8.58 (dd, 1H), 8.63 (d, 1H), 10.95 (s br, 1H), 11.52 (s br, 1H).

### Beispiel 3

### 2-[7-(2-Fluorbenzyl)imidazo[1,5-b]pyridazin-5-yl]-5,5-dimethyl-5,7-dihydro-6H-pyrrolo[2,3-d]pyrimidin-6-on

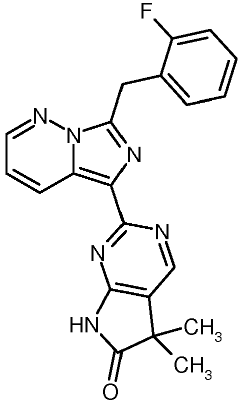

75 mg (0.146 mmol) Beispiel 1 wurden in Dimethylformamid (10 ml) mit 32 mg Palladium auf Aktivkohle (10%-ig) versetzt und 4 Stunden bei Wasserstoff-Normaldruck und 20°C hydriert. Nach Filtration über Celite wurde mittels präparativer HPLC gereinigt (Acetonitril:Wasser (+0.05 % Ameisensäure) - Gradient). Es wurden 30 mg der Zielverbindung erhalten (53 % d. Th.).

LC-MS (Methode 1): Rₜ = 0.86 min; MS (EIpos): m/z = 389 [M+H]⁺.

¹H-NMR (400 MHz, DMSO-d₆): δ [ppm] = 1.35 (s, 6H), 4.51 (s, 2H), 7.04 (dd, 1H), 7.12-7.23 (m, 2H), 7.28-7.35 (m, 2H), 8.46 (dd, 1H), 8.51 (s, 1H), 8.88 (dd, 1H), 11.45 (s br, 1H).

### Beispiel 4

### 3-[7-(2-Fluorbenzyl)imidazo[1,5-b]pyridazin-5-yl]-7,7-dimethyl-5,7-dihydro-6H-pyrrolo[2,3-e][1,2,4]triazin-6-on

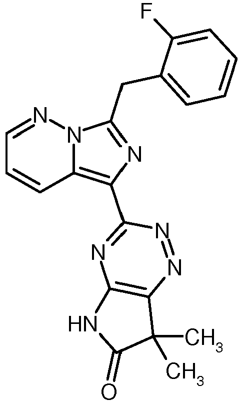

1.00 g (0.733 mmol) der Verbindung aus Beispiel 12A wurde mit 4.991 ml Phosphorylchlorid versetzt und über Nacht bei RT gerührt. Das Reaktionsgemisch wurde in 48 ml Acetonitril gegeben und unter Eiskühlung zu 30.29 ml konzentrierter wässriger Ammoniak-Lösung (33%-ig) getropft. Es wurde 3 Tage bei Raumtemperatur gerührt. Anschliessend wurde bis zur Trockene eingeengt. Der Rückstand wurde mit Ethanol verrührt, abgesaugt und mit Ethanol nachgewaschen. Das Filtrat wurde anschliessend bis zur Trockene eingeengt. Der Rückstand wurde in Wasser und Essigsäureethylester aufgenommen und die Phasen getrennt. Die organische Phase wurde viermal mit Wasser und anschliessend einmal mit gesättigter wässriger Natriumchloridlösung gewaschen und danach eingeengt. Es folgte eine Reinigung mittels präparativer HPLC (Acetonitril:Wasser (+0.05 % Ameisensäure) - Gradient). Es wurden 153 mg der Zielverbindung erhalten (51 % d. Th.).

LC-MS (Methode 3): Rₜ = 0.90 min; MS (EIpos): m/z = 390 [M+H]⁺.

¹H-NMR (400 MHz, DMSO-d₆): δ [ppm] = 1.43 (s, 6H), 4.54 (s, 2H), 7.09-7.23 (m, 3H), 7.26-7.35 (m, 2H), 8.52 (dd, 1H), 8.78 (dd, 1H), 12.04 (s br, 1H).

### Beispiel 5

### 2-[6-Chlor-3-(2,3,6-trifluorbenzyl)imidazo[1,5-a]pyridin-1-yl]-4-iod-5,5-dimethyl-5,7-dihydro-6H-pyrrolo[2,3-d]pyrimidin-6-on

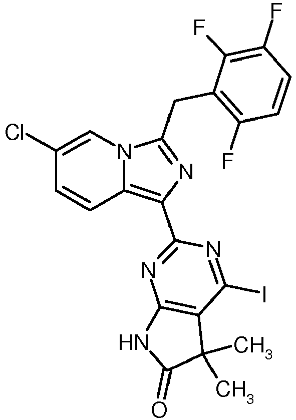

556 mg (1.176 mmol) Beispiel 15A wurden in 1,2-Dimethoxyethan (14 ml) vorgelegt und bei Raumtemperatur mit 305 mg (1.176 mmol) Cäsiumiodid, 149 mg (0.588 mmol) Iod und 67 mg (0.353 mmol) Kupfer-(I)-iodid versetzt. Anschliessend wurde iso-Pentylnitrit (0.933 ml) zugegeben und es wurde über Nacht auf 60°C erhitzt. Am nächsten Tag wurden nochmals 305 mg (1.176 mmol) Cäsiumiodid, 149 mg (0.588 mmol) Iod und 67 mg (0.353 mmol) Kupfer-(I)-iodid, sowie iso-Pentylnitrit (0.933 ml) zugegeben und es wurde für 3 Tage auf 60°C erhitzt. Nach Abkühlen wurde mit einem kleineren Ansatz vereinigt (ausgehend von 50 mg Beispiel 15A). Es wurde mit Essigsäureethylester und gesättigter wässriger Natriumthiosulfat extrahiert und die Phasen getrennt. Die organische Phase wurde noch zweimal mit gesättigter wässriger Natriumthiosulfat extrahiert. Anschliessend wurde die organische Phase mit gesättigter wässriger Natriumchloridlösung gewaschen, über Natriumsulfat getrocknet, filtriert, eingeengt und der Rückstand mittels präparativer HPLC gereinigt (Acetonitril:Wasser (+0.05 % Ameisensäure) - Gradient). Es wurden 236 mg der Titelverbindung erhalten (31% d. Th.).

LC-MS (Methode 1): Rt = 1.28 min; MS (ESIpos): m/z = 584 (M+H)⁺

¹H-NMR (400 MHz, DMSO-d₆): δ [ppm] = 1.37 (s, 6H), 4.57 (s, 2H), 7.19-7.25 (m, 1H), 7.30 (dd, 1H), 7.48-7.56 (m, 1H), 8.43 (d, 1H), 8.87 (s, 1H), 11.58 (s, 1H).

Neben der Titelverbindung wurden ausserdem 27 mg (5% d. Th., 90%ige Reinheit) 2-[6-Chlor-3-(2,3,6-trifluorbenzyl)imidazo[1,5-a]pyridin-1-yl]-4-hydroxy-5,5-dimethyl-5,7-dihydro-6H-pyrrolo[2,3-d]pyrimidin-6-on (Beispiel 6) erhalten.

### Beispiel 6

### 2-[6-Chlor-3-(2,3,6-trifluorbenzyl)imidazo[1,5-a]pyridin-1-yl]-4-hydroxy-5,5-dimethyl-5,7-dihydro-6H-pyrrolo[2,3-d]pyrimidin-6-on

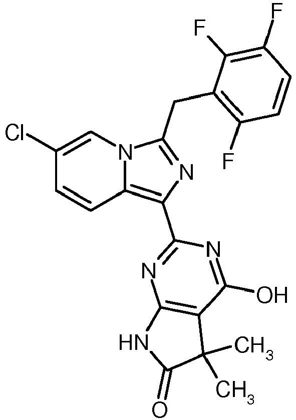

Entstanden als Nebenkomponente unter Beispiel 5. Ausbeute: 27 mg (5% d. Th., 90%ige Reinheit)

LC-MS (Methode 1): Rt = 0.97 min; MS (ESIpos): m/z = 474 (M+H)⁺

¹H-NMR (400 MHz, DMSO-d₆): δ [ppm] = 1.28 (s, 6H), 4.59 (s, 2H), 7.18-7.20 (m, 1H), 7.41 (d, 1H), 7.48-7.55 (m, 1H), 8.31 (d, 1H), 8.95 (s, 1H), 10.93 (s, 1H), 11.04 (s, 1H).

### Beispiel 7

### 2-[6-Chlor-3-(2,3,6-trifluorbenzyl)imidazo[1,5-a]pyridin-1-yl]-5,5-dimethyl-5,7-dihydro-6H-pyrrolo[2,3-d]pyrimidin-6-on

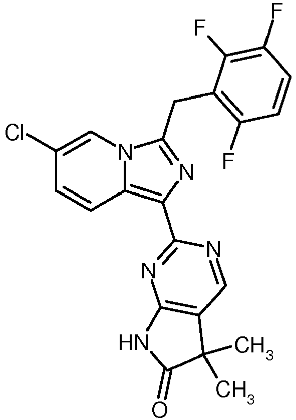

100 mg (0.171 mmol) von Beispiel 5 wurden in DMF (9 ml) gelöst zu 41.1 mg Palladium auf Kohle (10%-ig) in DMF (1 ml) gegeben und über Nacht bei Wasserstoff-Normaldruck hydriert. Anschliessend wurde über Celite filtriert, mit DMF gewaschen und bis zur Trockene eingeengt. Der Rückstand wurde mittels präparativer HPLC gereinigt (Acetonitril:Wasser (+0.05 % Ameisensäure) - Gradient).Es wurden 27 mg der Titelverbindung erhalten (34% d. Th., 96 %ige Reinheit).

LC-MS (Methode 2): Rₜ = 2.17 min; MS (ESIpos): m/z = 458 (M+H)⁺

¹H-NMR (400 MHz, DMSO-d₆): δ [ppm] = 1.33 (s, 6H), 4.56 (s, 2H), 7.17-7.25 (m, 2H), 7.48-7.56 (m, 1H), 8.46 (s, 1H), 8.51 (dd, 1H), 8.83 (s, 1H), 11.38 (s, 1H).

### Beispiel 8

### 2-[6-Fluor-3-(3,3,4,4,4-pentafluorbutyl)imidazo[1,5-a]pyridin-1-yl]-4-iod-5,5-dimethyl-5,7-dihydro-6H-pyrrolo[2,3-d]pyrimidin-6-on

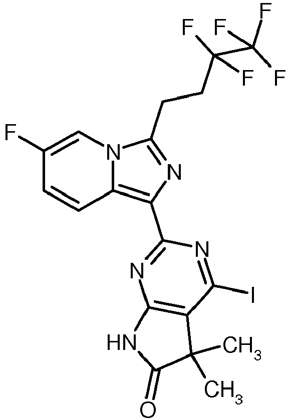

585 mg (1.276 mmol) Beispiel 19A wurden analog der Vorschrift unter Beispiel 5 umgesetzt. Nach Reinigung mittels präparativer HPLC (Acetonitril:Wasser (+0.05 % Ameisensäure) - Gradient) wurden 209 mg der Titelverbindung erhalten (28% d. Th.).

LC-MS (Methode 1): Rₜ = 1.22 min; MS (ESIpos): m/z = 570 (M+H)⁺

¹H-NMR (400 MHz, DMSO-d₆): δ [ppm] = 1.40 (s, 6H), 2.80-2.96 (m, 2H), 2H vermutlich unter Wassersignal, 7.34(t, 1H), 8.46 (dd, 1H), 8.73 (d, 1H), 11.62 (s, 1H).

Neben der Titelverbindung wurden ausserdem 89 mg (15% d. Th., 87%ige Reinheit) 2-[6-Fluor-3-(3,3,4,4,4-pentafluorbutyl)imidazo[1,5-a]pyridin-1-yl]-4-hydroxy-5,5-dimethyl-5,7-dihydro-6H-pyrrolo[2,3-d]pyrimidin-6-on (Beispiel 9) erhalten.

### Beispiel 9

### 2-[6-Fluor-3-(3,3,4,4,4-pentafluorbutyl)imidazo[1,5-a]pyridin-1-yl]-4-hydroxy-5,5-dimethyl-5,7-dihydro-6H-pyrrolo[2,3-d]pyrimidin-6-on

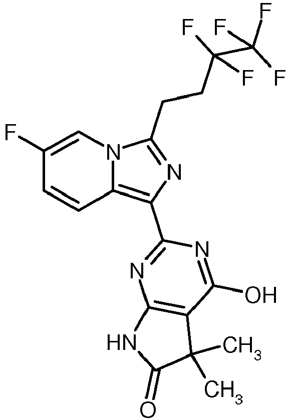

Entstanden als Nebenkomponente unter Beispiel 8. Ausbeute: 89 mg (15% d. Th., 87%ige Reinheit)

LC-MS (Methode 1): Rₜ = 0.96 min; MS (ESIpos): m/z = 460 (M+H)⁺

¹H-NMR (400 MHz, DMSO-d₆): δ [ppm] = 1.31 (s, 6H), 2.88-3.07 (m, 2H), 2H vermutlich unter Wassersignal, 7.45(t, 1H), 8.32 (dd, 1H), 8.81 (d, 1H), 10.91 (s, 1H), 11.56 (s, 1H).

### Beispiel 10

### 2-[6-Fluor-3-(3,3,4,4,4-pentafluorbutyl)imidazo[1,5-a]pyridin-1-yl]-5,5-dimethyl-5,7-dihydro-6H-pyrrolo[2,3-d]pyrimidin-6-on

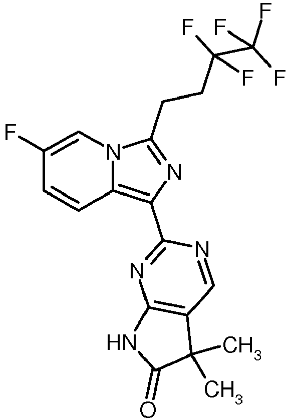

100 mg (0.176 mmol) von Beispiel 8 wurden in Analogie zur Vorschrift unter Beispiel 7 hydriert. Es wurden 24 mg der Titelverbindung erhalten (30% d. Th.).

LC-MS (Methode 3): Rt = 0.95 min; MS (ESIpos): m/z = 444 (M+H)⁺

¹H-NMR (400 MHz, DMSO-d₆): δ [ppm] = 1.36 (s, 6H), 2.84-2.95 (m, 2H), 2H vermutlich unter Wassersignal, 7.20-7.25 (m, 1H), 8.50 (s, 1H), 8.55 (dd, 1H), 8.68 (dd, 1H), 11.43 (s, 1H).

### Beispiel 11

### 3-[6-Chlor-3-(2,3,6-trifluorbenzyl)imidazo[1,5-a]pyridin-1-yl]-7,7-dimethyl-5,7-dihydro-6H-pyrrolo[2,3-e][1,2,4]triazin-6-on

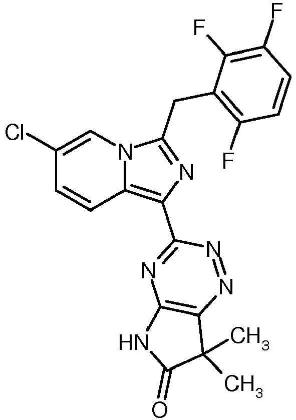

1.665 g (ca. 2.407 mmol, 71%ige Reinheit) der Verbindung aus Beispiel 21A wurden mit 16 ml Phosphorylchlorid versetzt und über Nacht bei RT gerührt. Das Reaktionsgemisch wurde in 160 ml Acetonitril gelöst und unter Eiskühlung in 100 ml konzentrierte wässriger Ammoniak-Lösung (33%-ig) eingerührt. Es wurde 3 Tage bei RT gerührt. Anschliessend wurde das Reaktionsgemisch eingeengt. Der Rückstand wurde in Wasser und Ethanol aufgenommen und 1h bei RT gerührt. Der entstandene Niederschlag wurde abgesaugt und mit Wasser und wenig Ethanol nachgewaschen. Man erhielt nach Trocknung im Hochvakuum 899 mg der Titelverbindung (63 % d. Th., 78%ige Reinheit). 700 mg dieses Produktes wurden mittels präparativer HPLC gereinigt (Acetonitril:Wasser (+0.05 % Ameisensäure) - Gradient). Es wurden 212 mg der Titelverbindung erhalten (19 % d. Th.).

LC-MS (Methode 3): Rt = 1.03 min; MS (EIpos): m/z = 459 [M+H]⁺.

¹H-NMR (400 MHz, DMSO-d₆): δ [ppm] = 1.40 (s, 6H), 4.58 (s, 2H), 7.20-7.29 (m, 2H), 7.48-7.56 (m, 1H), 8.47 (d, 1H), 8.88 (s, 1H), 11.96 (br s, 1H).

### Beispiel 12

### 3-[6-Fluor-3-(3,3,4,4,4-pentafluorbutyl)imidazo[1,5-a]pyridin-1-yl]-7,7-dimethyl-5,7-dihydro-6H-pyrrolo[2,3-e][1,2,4]triazin-6-on

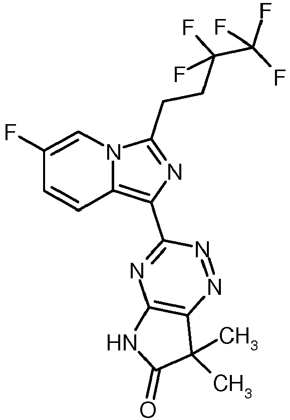

1.490 g (ca. 2.026 mmol, 65%ige Reinheit) der Verbindung aus Beispiel 23A wurden mit 13 ml Phosphorylchlorid versetzt und über Nacht bei RT gerührt. Das Reaktionsgemisch wurde in 130 ml Acetonitril gelöst und unter Eiskühlung in 85 ml konzentrierte wässriger Ammoniak-Lösung (33%-ig) eingerührt. Es wurde 3 Tage bei RT gerührt. Anschliessend wurde das Reaktionsgemisch eingeengt. Der Rückstand wurde in Wasser und Ethanol aufgenommen und 1h bei RT gerührt. Der entstandene Niederschlag wurde abgesaugt und mit Wasser und wenig Ethanol nachgewaschen. Man erhielt nach Trocknung im Hochvakuum 839 mg der Titelverbindung (62 % d. Th., 67%ige Reinheit). 200 mg dieses Produktes wurden mittels präparativer HPLC gereinigt (Acetonitril:Wasser (+0.05 % Ameisensäure) - Gradient). Es wurden 71 mg der Titelverbindung erhalten (7 % d. Th.).

LC-MS (Methode 1): Rt = 0.95 min; MS (EIpos): m/z = 445 [M+H]⁺.

¹H-NMR (400 MHz, DMSO-d₆): δ [ppm] = 1.43 (s, 6H), 2.83-2.96 (m, 2H), 2H vermutlich unter Wassersignal, 7.29-7.34 (m, 1H), 8.51 (dd, 1H), 8.74 (dd, 1H), 12.01 (br s, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 3 | 72 |
| 4 | 42 |
| 11 | 30 |
| 12 | 276 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.1 |
| 3 | 0.03 |
| 4 | 0.03 |
| 7 | 0.01 |
| 11 | 0.03 |
| 12 | 1.0 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

### Das System besteht aus 3 Hauptkomponenten:

### Implantierbare Sender (Physiotel® Telemetrietransmitter)

Empfänger (Physiotel® Receiver), die über einen Multiplexer (DSI Data Exchange Matrix) mit einem

Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p. ) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

### Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI ) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
Systolischer Blutdruck (SBP)
Diastolischer Blutdruck (DBP)
Arterieller Mitteldruck (MAP)
Herzfrequenz (HR)
Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-4. Bestimmung pharmakokinetischer Kenngrößen nach intravenöser und oraler Gabe

Die pharmakokinetischen Parameter der erfindungsgemäßen Verbindungen der Formel (I) werden in männlichen Wister-Ratten bestimmt. Das Applikationsvolumen beträgt 5 mL/kg. Die intravenöse Gabe erfolgt mittels einer speziesspezifischen Plasma/DMSO-Formulierung (99/1). Den Ratten wird zur vereinfachten Blutabnahme vor der Substanzgabe ein Silikonkatheter in die rechte *Vena jugularis externa* gelegt. Die Operation erfolgt einen Tag vor dem Versuch unter Isofluran-Narkose und unter Gabe eines Analgetikums (Atropin/Rimadyl (3/1) 0.1 mL s.c.). Die Substanzgabe wird i.v. bolus durchgeführt. Die Blutabnahme erfolgt bei Ratten nach 0.033, 0.083, 0.17, 0.5, 1, 2, 3, 4, 6, 8, 24 und 27 Stunden. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen der Formel (I), Kalibrierproben und QC's wird ein interner Standard zugesetzt (ZK 228859) und es folgt eine Proteinfällung mittels Acetonitril im Überschuss. Nach Zugabe eines Ammoniumacetat-Puffers (0.01 M, pH 6.8) und folgendem Vortexen wird bei 1000 g zentrifugiert und der Überstand mittels LC-MS/MS (API 4000, AB Sciex) vermessen. Die chromatographische Trennung wird auf einer 1100-HPLC von Agilent durchgeführt. Das Injektionsvolumen beträgt 20 µL. Als Trennsäule wird eine auf 40°C temperierte Gemini 5µ C18 110A 50x3mm der Fa. Phenomenex verwendet. Es wird ein binärer Laufmittelgradient bei 500 µL/min gefahren (A: 0.01M Ammoniumacetat-Puffer pH 6.8, B: 0.1 % Ameisensäure in Acetonitril): 0 Min. (90 % A), 1 Min. (90 % A), 3 Min. (10 % A), 4 Min. (10 % A), 4.50 Min. (90 % A), 6 Min. (90 % A). Die Temperatur der Turbo V Ionenquelle beträgt 400°C. Folgende MS-Geräteparameter werden benutzt: Curtain Gas 15 units, Ionsprayvoltage 4.8 kV, Gas 1 50 units, Gas 2 40 units, CAD Gas 8 units. Die Quantifizierung der Substanzen erfolgt anhand der Peakhöhen oder -flächen aus extrahierten Ionenchromatogrammen spezifischer MRM-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels des validierten pharmakokinetischen Rechenprogramms KinEx (Vers.3) berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (s.o.) und durch Quotientenbildung der C_{Blut}/C_{Plasma}-Wert ermittelt.

Nach intravenöser Gabe von 0.3 mg/kg repräsentativer erfindungsgemäßer Verbindungen in Ratten wurden die in Tabelle 3 aufgeführten Werte erhoben:

**Tabelle 3:**

| | | |
|---|---|---|
| Beispiel | 11 | 12 |
| CL _{Blut} [L/h/kg] | 0.03 | 0.16 |
| Terminale Halbwertszeit [h] | 7.2 | 5.2 |
| Mean Residence Time [h] | 9.4 | 4.6 |

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mm Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 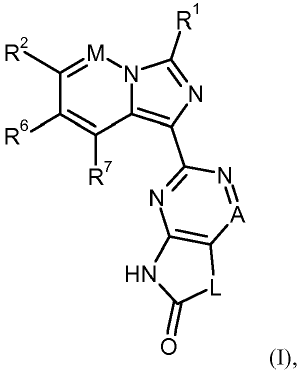 2. in welcher
A für Stickstoff oder CR³ steht,
wobei
R³ für Wasserstoff steht,
L für eine Gruppe #-CR^{4A}R^{4B}-(CR^{5A}R^{5B})p-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
M für CH oder N steht,
R¹ für 4,4,4-Trifluorbut-1-yl, 3,3,4,4-Tetrafluorbut-1-yl, 3,3,4,4,4-Pentafluorbut-1-yl oder Benzyl steht,
wobei Benzyl mit 1 bis 3 Substituenten Fluor substituiert ist,
R² für Fluor oder Chlor steht, wenn M für CH steht,
oder
R² für Wasserstoff steht, wenn M für N steht,
R⁶ für Wasserstoff steht,
R⁷ für Wasserstoff steht,
sowie ihre Salze, Solvate und Solvate der Salze.

2. Verfahren zur Herstellung von Verbindungen der Formel (I), wie in Anspruch 1 den Ansprüchen 1 bis 4 definiert, **dadurch gekennzeichnet, dass** man
[A] eine Verbindung der Formel (II) 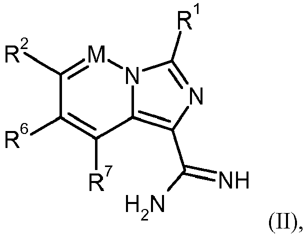 in welcher M, R¹, R², R⁶ und R⁷ jeweils die in Anspruch 1 angegebenen Bedeutungen haben,
in einem inerten Lösungsmittel in Gegenwart einer geeigneten Base mit einer Verbindung der Formel (III) 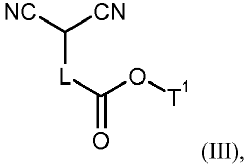
in welcher L die in Anspruch 1 angegebene Bedeutung hat und
T¹ für (C₁-C₄)-Alkyl steht,
zu einer Verbindung der Formel (IV) 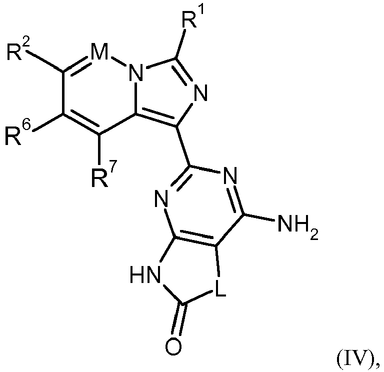 in welcher M, L, R¹, R², R⁶ und R⁷ jeweils die in Anspruch 1 angegebenen Bedeutungen haben,
cyclisiert, diese anschließend in einem inerten Lösungsmittel mit iso-Pentylnitrit und einem Halogen-Äquivalent in eine Verbindung der Formel (I-A) 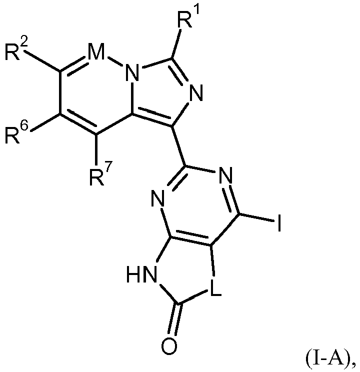 in welcher M, L, R¹, R², R⁶ und R⁷ jeweils die in Anspruch 1 angegebenen Bedeutungen haben,
überführt,
oder
[B] eine Verbindung der Formel (I-A) in einen inerten Lösungsmittel in Gegenwart eines geeigneten Übergangsmetallkatalysators zu einer Verbindung der Formel (I-B) 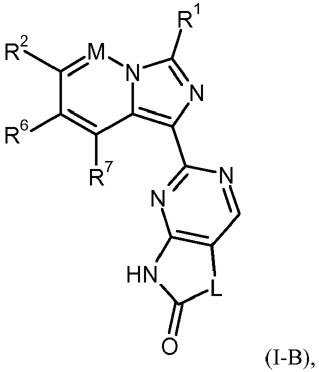 in welcher M, L, R¹, R², R⁶ und R⁷ jeweils die in Anspruch 1 angegebenen Bedeutungen haben,
umsetzt,
oder
[C] eine Verbindung der Formel (II) in einem inerten Lösungsmittel in Gegenwart einer geeigneten Base mit einer Verbindung der Formel (V) 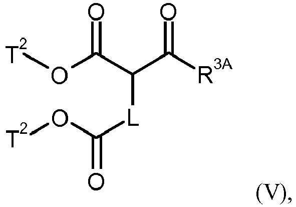 in welcher L die in Anspruch 1 angegebene Bedeutung hat,
R^{3A} für Wasserstoff, Halogen, (C₁-C₄)-Alkyl oder Hydroxy steht und
T² für (C₁-C₄)-Aklyl steht,
zu einer Verbindung der Formel (VI) 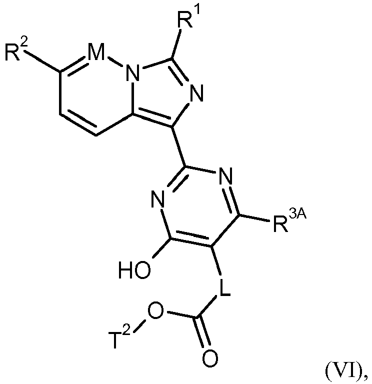 in welcher M, L, R¹, R², R^{3A}, R⁶, R⁷ und T² jeweils die zuvor angegebenen Bedeutungen haben,
umsetzt, diese anschliessend mit Phosphorylchlorid in eine Verbindung der Formel (VII) 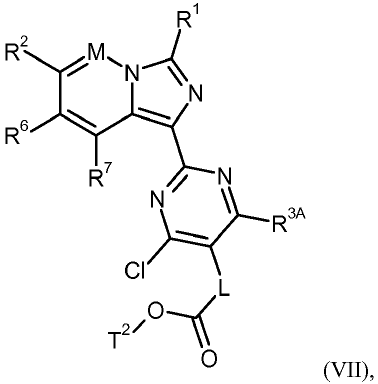 in welcher M, L, R¹, R², R^{3A}, R⁶, R⁷ und T² jeweils die zuvor angegebenen Bedeutungen haben,
überführt, diese im folgenden in einem inerten Lösungsmittel in eine entsprechende Azid-Verbindung überführt und diese direkt zu einer Verbindung der Formel (VIII) 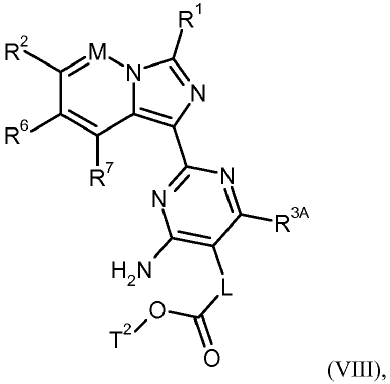 in welcher M, L, R¹, R², R^{3A}, R⁶, R⁷ und T² jeweils die zuvor angegebenen Bedeutungen haben,
reduziert, und diese dann in einem inerten Lösungsmittel in Gegenwart einer geeigneten Base zu einer Verbindung der Formel (I-C) 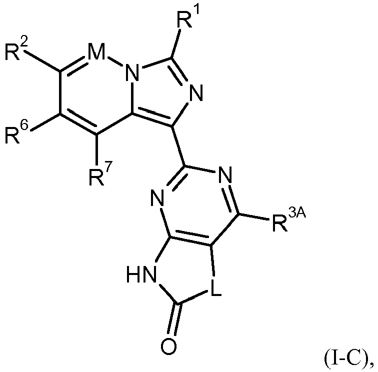 in welcher M, L, R¹, R² und R^{3A}, R⁶, R⁷ jeweils die zuvor angegebenen Bedeutungen haben,
umsetzt,
oder
[D] eine Verbindung der Formel (II) in einem inerten Lösungsmittel in Gegenwart einer geeigneten Base mit Hydrazinhydrat zu einer Verbindung der Formel (IX) 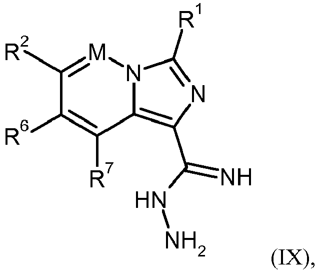 in welcher M, R¹, R², R⁶ und R⁷ jeweils die in Anspruch 1 angegebenen Bedeutungen haben,
umsetzt, diese dann in einem inerten Lösungsmittel mit einer Verbindung der Formel (X) 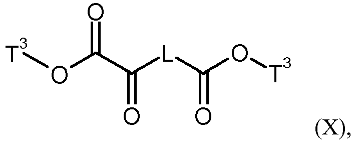
in welcher L die in Anspruch 1 angegebene Bedeutung hat und
T³ für (C₁-C₄)-Alkyl steht,
zu einer Verbindung der Formel (XI) 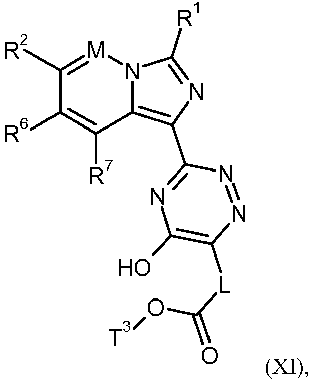 in welcher M, L, R¹, R², R⁶, R⁷ und T³ jeweils die in Anspruch 1 angegebenen Bedeutungen haben,
reagiert, diese anschliessend in eine Verbindung der Formel (XII) 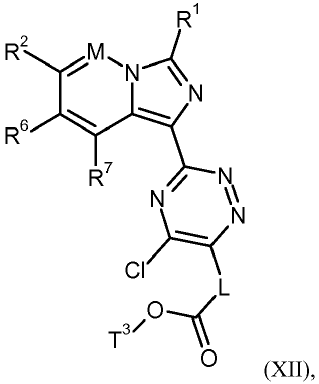 in welcher M, L, R¹, R², R⁶, R⁷ und T³ jeweils die in Anspruch 1 angegebenen Bedeutungen haben,
überführt, und diese direkt mit Ammoniak zu einer Verbindung der Formel (XIII) 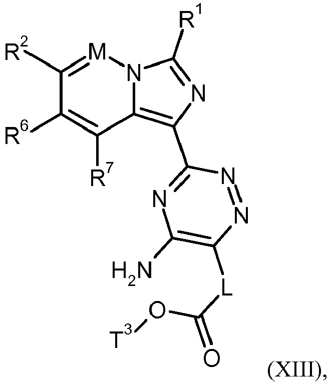 in welcher M, L, R¹, R², R⁶, R⁷ und T³ jeweils die in Anspruch 1 angegebenen Bedeutungen haben,
umsetzt und schliesslich in einem inerten Lösungsmittel gegebenenfalls in Gegenwart einer geeigneten Base zu einer Verbindung der Formel (I-D) 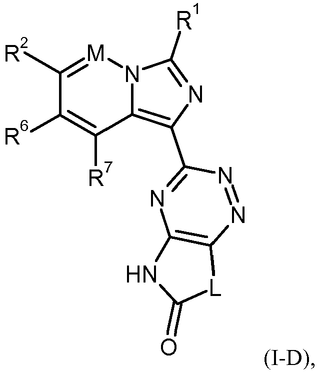 in welcher M, L, R¹, R², R⁶ und R⁷ jeweils die in Anspruch 1 angegebenen Bedeutungen haben"
cyclisiert,
gegebenenfalls die resultierenden Verbindungen der Formel (I-A), (I-B), (I-C) und (I-D) mit den entsprechenden (i) Lösungsmitteln und/oder (ii) Säuren oder Basen in ihre Solvate, Salze und/oder Solvate der Salze überführt.

3. Verbindung der Formel (I), wie in Anspruch 1 definiert, zur Verwendung in einem Verfahren zur Behandlung und/oder Prophylaxe von Krankheiten.

4. Verwendung einer Verbindung der Formel (I), wie in Anspruch 1 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

5. Verbindung der Formel (I), wie in Anspruch 1 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

6. Arzneimittel enthaltend eine Verbindung der Formel (I), wie in Anspruch 1 definiert, in Kombination mit einem inerten, nicht-toxischen, pharmazeutisch geeigneten Hilfsstoff.

7. Arzneimittel enthaltend eine Verbindung der Formel (I), wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

8. Arzneimittel nach Anspruch 6 oder 7 zur Behandlung und/oder Prophylaxe von Herz-insuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 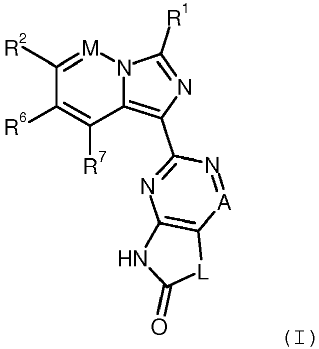 in which
A is nitrogen or CR³,
where
R³ represents hydrogen,
L is a #-CR^{4A}R^{4B}-(CR^{5A}R^{5B})ₚ-## group
where
# is the attachment site to the carbonyl group,
## is the point of attachment to the pyrimidine or triazine ring,
p is a number 0,
R^{4A} is hydrogen, fluorine, methyl or hydroxy,
R^{4B} is hydrogen, fluorine, trifluoromethyl, 2,2,2-trifluoroethyl or methyl,
M is CH or N,
R¹ is 4,4,4-trifluorobut-1-yl, 3,3,4,4-tetrafluorobut-1-yl, 3,3,4,4,4-pentafluorobut-1-yl or benzyl, where benzyl is substituted by 1 to 3 fluorine substituents,
R² is fluorine or chlorine if M is CH,
or R² is hydrogen if M is N,
R⁶ is hydrogen,
R⁷ represents hydrogen,
and the salts, solvates and solvates of the salts thereof.

2. Process for preparing compounds of the formula (I) as defined in Claim 1, **characterized in that**
[A] a compound of the formula (II) 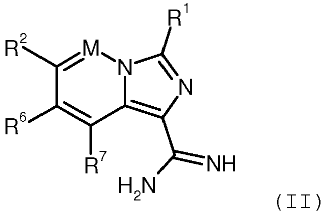 in which M, R¹, R², R⁶ and R⁷ each have the meanings given in Claim 1,
are cyclized in an inert solvent in the presence of a suitable base with a compound of the formula (III) 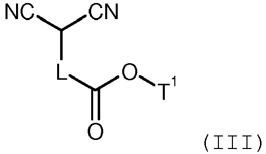
in which L has the meaning given in Claim 1 and
T¹ is (C₁-C₄) -alkyl
to give a compound of the formula (IV) 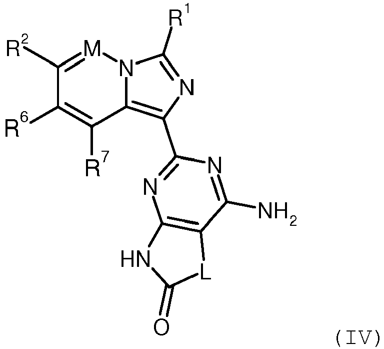 in which M, L, R¹, R², R⁶ and R⁷ each have the meanings given in Claim 1,
this is then converted in an inert solvent using isopentyl nitrite and a halogen equivalent into a compound of the formula (I-A) 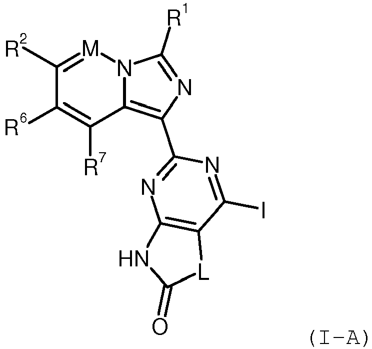 in which M, L, R¹, R², R⁶ and R⁷ each have the meanings given in Claim 1,
or
[B] a compound of the formula (I-A) is reacted in an inert solvent in the presence of a suitable transition metal catalyst to give a compound of the formula (I-B) 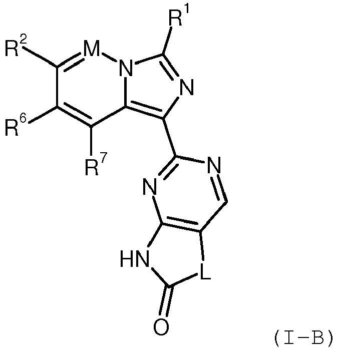 in which M, L, R¹, R², R⁶ and R⁷ each have the meanings given in Claim 1,
or
[C] a compound of the formula (II) is reacted in an inert solvent in the presence of a suitable base with a compound of the formula (V) 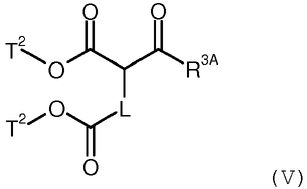
in which L has the meaning given in Claim 1,
R^{3A} is hydrogen, halogen, (C₁-C₄)-alkyl or hydroxy and
T² is (C₁-C₄)-alkyl,
to give a compound of the formula (VI) 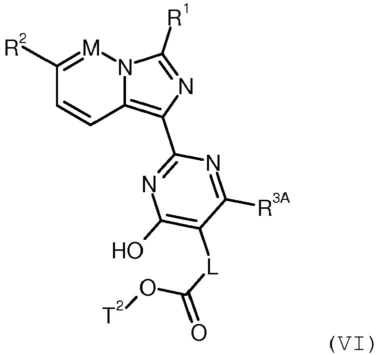 in which M, L, R¹, R², R^{3A}, R⁶, R⁷ and T² each have the meanings given above,
this is then converted with phosphoryl chloride into a compound of the formula (VII) 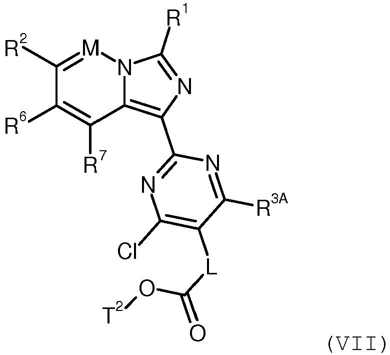 in which M, L, R¹, R², R^{3A}, R⁶, R⁷ and T² each have the meanings given above,
this is subsequently converted in an inert solvent into a corresponding azide compound and this is reduced directly to give a compound of the formula (VIII) 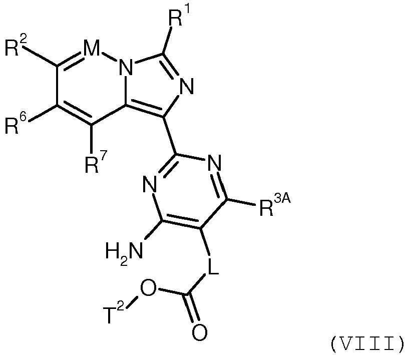 in which M, L, R¹, R², R^{3A}, R⁶, R⁷ and T² each have the meanings given above,
and this is then reacted in an inert solvent, in the presence of a suitable base to give a compound of the formula (I-C) 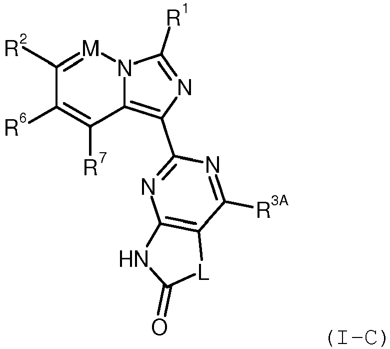 in which M, L, R¹, R² and R^{3A}, R⁶, R⁷ each have the meanings given above,
or
[D] a compound of the formula (II) is reacted in an inert solvent in the presence of a suitable base with hydrazine hydrate to give a compound of the formula (IX) 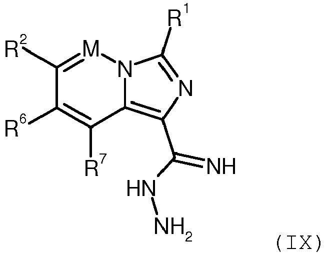 in which M, R¹, R², R⁶ and R⁷ each have the meanings given in Claim 1,
this is then reacted in an inert solvent with a compound of the formula (X) 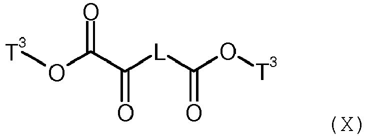
in which L has the meaning given in Claim 1 and
T³ is (C₁-C₄) -alkyl
to give a compound of the formula (XI) 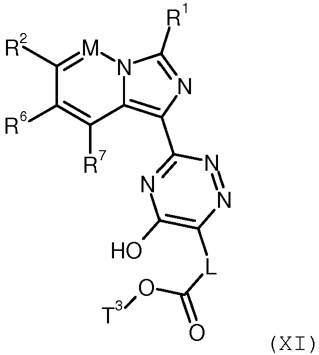 in which M, L, R¹, R², R⁶, R⁷ and T³ each have the meanings given in Claim 1,
this is then converted into a compound of the formula (XII) 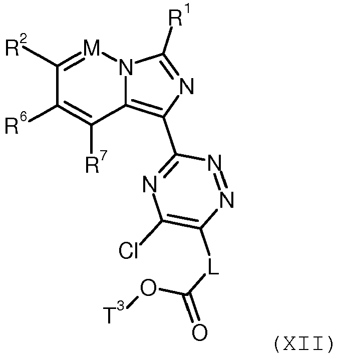 in which M, L, R¹, R², R⁶, R⁷ and T³ each have the meanings given in Claim 1,
and this is reacted directly with ammonia to give a compound of the formula (XIII) 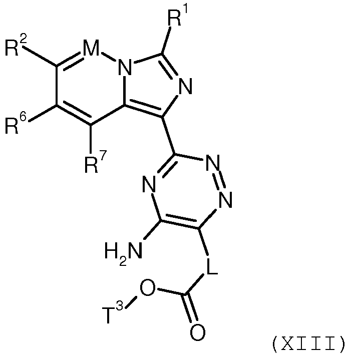 in which M, L, R¹, R², R⁶, R⁷ and T³ each have the meanings given in Claim 1,
and finally cyclized in an inert solvent, optionally in the presence of a suitable base, to give a compound of the formula (I-D) 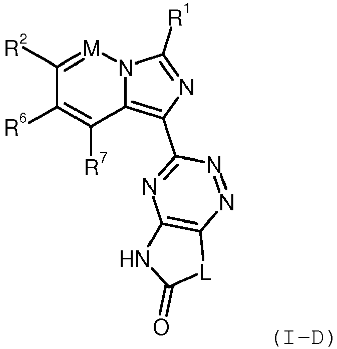 in which M, L, R¹, R², R⁶ and R⁷ each have the meanings given in Claim 1,
and the resulting compounds of the formulae (I-A), (I-B), (I-C) and (I-D) with the optionally the resulting compounds of the formula (I) are, where appropriate, converted with the appropriate (i) solvents and/or (ii) acids or bases into their solvates, salts and/or solvates of the salts.

3. Compound of the formula (I) as defined in Claim 1 for use in a method for the treatment and/or prophylaxis of diseases.

4. Use of a compound of the formula (I) as defined in Claim 1 for production of a medicament for treatment and/or prophylaxis of heart failure, angina pectoris, hypertension, pulmonary hypertension, ischemia, vascular disorders, kidney failure, thromboembolic disorders, fibrotic disorders and arteriosclerosis.

5. Compound of the formula (I) as defined in Claim 1 for use in a method for the treatment and/or prophylaxis of heart failure, angina pectoris, hypertension, pulmonary hypertension, ischemias, vascular disorders, kidney failure, thromboembolic disorders, fibrotic disorders and arteriosclerosis.

6. Medicament comprising a compound of the formula (I) as defined in Claim 1 in combination with an inert, nontoxic, pharmaceutically suitable excipient.

7. Medicament comprising a compound of the formula (I) as defined in Claim 1 in combination with a further active compound selected from the group consisting of organic nitrates, NO donors, cGMP-PDE inhibitors, antithrombotic agents, hypotensive agents and lipid metabolism modifiers.

8. Medicament according to Claim 6 or 7 for the treatment and/or prophylaxis of heart failure, angina pectoris, hypertension, pulmonary hypertension, ischemias, vascular disorders, kidney failure, thromboembolic disorders, fibrotic disorders and arteriosclerosis.

## Revendications

1. Composé de formule (I) 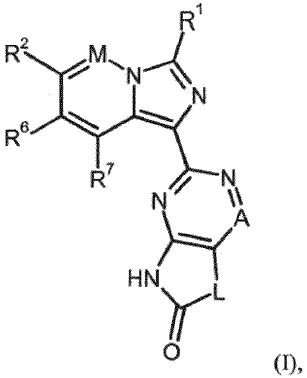 dans laquelle
A représente azote ou CR³,
R³ représentant hydrogène,
L représente un groupe #-CR^{4A}R^{4B}- (CR^{5A}R^{5B})ₚ-##,
# représentant l'emplacement de liaison au groupe carbonyle,
## représentant l'emplacement de liaison au cycle pyrimidine ou triazine,
p représentant le nombre 0,
R^{4A} représentant hydrogène, fluor, méthyle ou hydroxy,
R^{4B} représentant hydrogène, fluor, trifluorométhyle, 2,2,2-trifluoroéthyle ou méthyle,
M représente CH ou N,
R¹ représente 4,4,4-triflurobut-1-yle, 3,3,4,4-tétrafluorobut-1-yle, 3,3,4,4,4-pentafluorobut-1-yle ou benzyle, benzyle étant substitué avec 1 à 3 substituants fluor,
R² représente fluor ou chlore lorsque M représente
CH, ou
R² représente hydrogène lorsque M représente N,
R⁶ représente hydrogène,
R⁷ représente hydrogène,
ainsi que leurs sels, solvates et solvates des sels.

2. Procédé de fabrication de composés de formule (I), tels que définis dans la revendication 1, **caractérisé en ce que**
[A] un composé de formule (II) 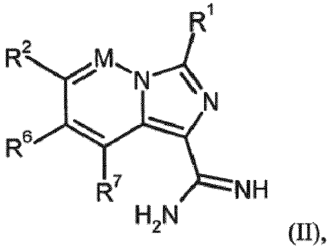 dans laquelle M, R¹, R², R⁶ et R⁷ ont chacun les significations indiquées dans la revendication 1, est cyclisé dans un solvant inerte en présence d'une base appropriée avec un composé de formule (III) 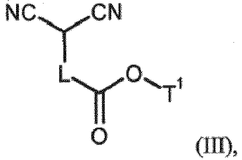
dans laquelle L a la signification indiquée dans la revendication 1, et
T¹ représente alkyle en (C₁-C₄),
pour former un composé de formule (IV) 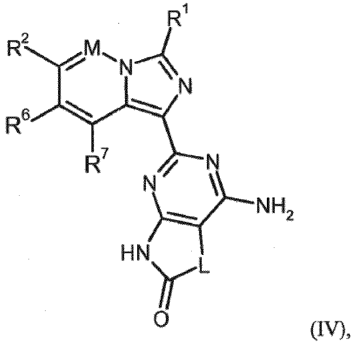 dans laquelle M, L, R¹, R², R⁶ et R⁷ ont les significations indiquées dans la revendication 1,
puis celui-ci est transformé dans un solvant inerte avec de l'iso-pentylnitrite et un équivalent d'halogène en un composé de formule (I-A) 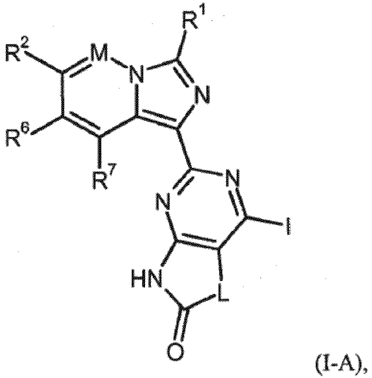 dans laquelle M, L, R¹, R², R⁶ et R⁷ ont chacun les significations indiquées dans la revendication 1,
ou
[B] un composé de formule (I-A) est mis en réaction dans un solvant inerte en présence d'un catalyseur de métal de transition approprié pour former un composé de formule (I-B) 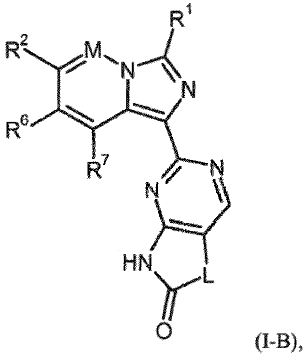 dans laquelle M, L, R¹, R², R⁶ et R⁷ ont chacun les significations indiquées dans la revendication 1,
ou
[C] un composé de formule (II) est mis en réaction dans un solvant inerte en présence d'une base appropriée avec un composé de formule (V) 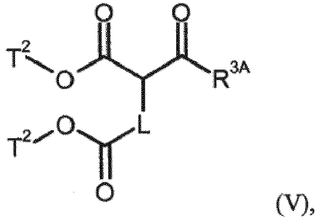 dans laquelle L a la signification indiquée dans la revendication 1,
R^{3A} représente hydrogène, halogène, alkyle en (C₁-C₄) ou hydroxy, et
T² représente alkyle en (C₁-C₄),
pour former un composé de formule (VI) 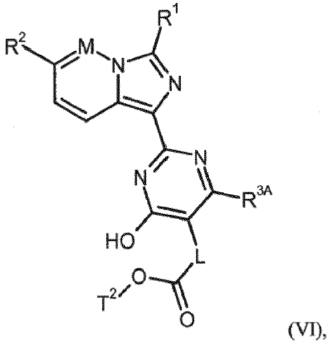 dans laquelle M, L, R₁, R₂, R^{3A}, R⁶, R⁷ et T² ont chacun les significations indiquées précédemment,
puis celui-ci est transformé avec du chlorure de phosphoryle en un composé de formule (VII) 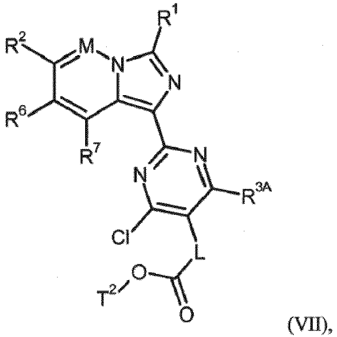 dans laquelle M, L, R¹, R², R^{3A}, R⁶, R⁷ et T² ont chacun les significations indiquées précédemment,
puis celui-ci est transformé dans un solvant inerte en un composé d'azide correspondant et celui-ci est réduit directement en un composé de formule (VIII) 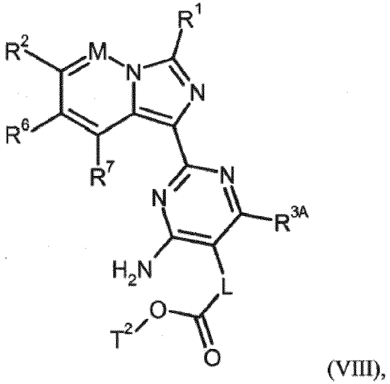 dans laquelle M, L, R¹, R², R^{3A}, R⁶, R⁷ et T² ont chacun les significations indiquées précédemment,
puis celui-ci est mis en réaction dans un solvant inerte en présence d'une base appropriée pour former un composé de formule (I-C) 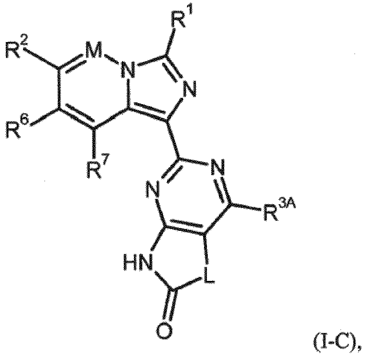 dans laquelle M, L, R¹, R² et R^{3A}, R⁶, R⁷ ont chacun les significations indiquées précédemment,
ou
[D] un composé de formule (II) est mis en réaction dans un solvant inerte en présence d'une base appropriée avec de l'hydrate d'hydrazine pour former un composé de formule (IX) 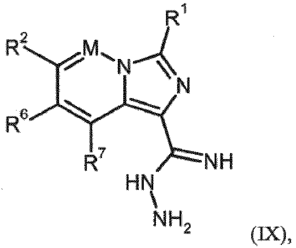 dans laquelle M, R¹, R², R⁶ et R⁷ ont chacun les significations indiquées dans la revendication 1,
puis celui-ci est mis en réaction dans un solvant inerte avec un composé de formule (X) 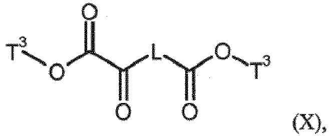
dans laquelle L a la signification indiquée dans la revendication 1, et
T³ représente alkyle en (C₁-C₄),
pour former un composé de formule (XI) 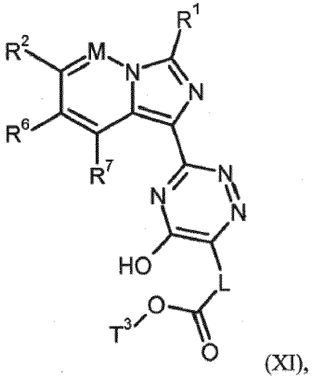 dans laquelle M, L, R¹, R², R⁶, R⁷ et T³ ont chacun les significations indiquées dans la revendication 1,
puis celui-ci est transformé en un composé de formule (XII) 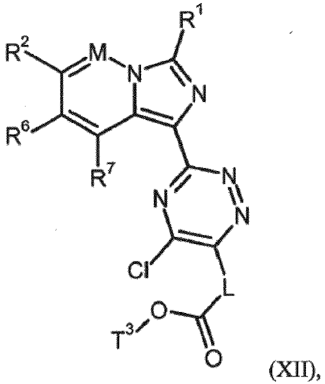 dans laquelle M, L, R¹, R², R⁶, R⁷ et T³ ont chacun les significations indiquées dans la revendication 1,
et celui-ci est directement mis en réaction avec de l'ammoniac pour former un composé de formule (XIII) 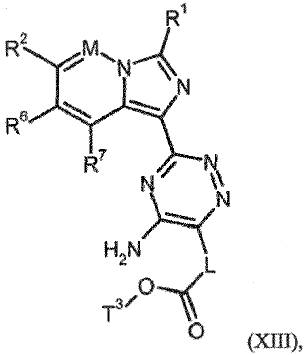 dans laquelle M, L, R¹, R², R⁶, R⁷ et T³ ont chacun les significations indiquées dans la revendication 1,
puis cyclisé dans un solvant inerte éventuellement en présence d'une base appropriée en un composé de formule (I-D) 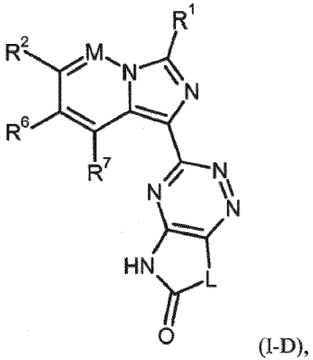 dans laquelle M, L, R¹, R², R⁶ et R⁷ ont chacun les significations indiquées dans la revendication 1,
les composés de formule (I-A), (I-B), (I-C) et (I-D) résultants avec les éventuellement les composés de formule (I) résultants étant éventuellement transformés avec (i) les solvants et/ou (ii) les acides ou les bases appropriés en leurs solvates, sels et/ou solvates des sels.

3. Composé de formule (I), tel que défini dans la revendication 1, destiné à une utilisation dans un procédé de traitement et/ou de prophylaxie de maladies.

4. Utilisation d'un composé de formule (I), tel que défini dans la revendication 1, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

5. Composé de formule (I), tel que défini dans la revendication 1, destiné à une utilisation dans un procédé de traitement et/ou de prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

6. Médicament contenant un composé de formule (I), tel que défini dans la revendication 1, en combinaison avec un adjuvant inerte, non toxique, pharmaceutiquement approprié.

7. Médicament contenant un composé de formule (I), tel que défini dans la revendication 1, en combinaison avec un autre agent actif choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

8. Médicament selon la revendication 6 ou 7, pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.
